# FASCICULE DE BREVET EUROPEEN

(11) **EP 4 340 905 B1**
(45) Date de publication et mention de la délivrance du brevet: **26.03.2025**
(21) Numéro de dépôt: 22731745.0
(22) Date de dépôt: 20.05.2022
(51) Int. Cl.: A61M 1/16, A61M 1/34, A61M 39/10, A61M 39/20

(54) **MACHINE DE DIALYSE ET PROCÉDÉ CORRESPONDANT**
DIALYSEMASCHINE UND ENTSPRECHENDES VERFAHREN
DIALYSIS MACHINE AND CORRESPONDING METHOD

(30) Priorité: 21.05.2021 FR 2105339; 21.05.2021 FR 2105337
(43) Date de publication de la demande: 27.03.2024
(73) Titulaire: Physidia, 49124 Saint-Barthelemy-D'Anjou (FR)
(72) Inventeur: TODOROVA, Véliana, 69140 Rillieux la Pape (FR); VINCENT, Eric, 49000 Angers (FR)
(74) Mandataire: Ipsilon
(86) Numéro de dépôt international: PCT/FR2022/050968
(87) Numéro de publication internationale: WO 2022/243647

(56) Documents cités:
- WO-A1-2008/035422
- WO-A2-2019/150058
- US-A1- 2013 237 903
- US-A1- 2014 316 332
- US-A1- 2016 243 347
- US-A1- 2017 072 122
- US-A1- 2020 230 301
- US-A1- 2021 100 946

## Description

### DOMAINE DE L'INVENTION

La présente invention concerne de manière générale les machines de dialyse, notamment pour l'hémodialyse, ainsi que les dispositifs pour machine de dialyse et l'utilisation (fonctionnement et manipulation) de tout ou partie de telles machines.

### ART ANTERIEUR

On connaît de l'état de la technique des machines de dialyse, notamment telles que décrites dans la demande WO2019150058A2.

Dans l'exemple de la demande WO2019150058A2, et comme illustré dans la présente demande en Figure 1, un système de raccordement 450E permet de raccorder une source d'alimentation en dialysat au système délivrance de dialysat.

Le système de raccordement 450E se présente sous la forme d'une tubulure en Y dont une première branche 451E permet la connexion à une source d'alimentation en dialysat pour une première séance de dialyse, et une deuxième branche 452E permet la connexion à une nouvelle source d'alimentation en dialysat pour une deuxième séance de dialyse. Les branches 451E et 452E se rejoignent au niveau d'une conduite 455E, formant le pied du Y, qui est en communication fluidique avec le système de délivrance en dialysat.

Ce système de raccordement permet de limiter le risque de contamination en permettant d'utiliser une branche pour la première séance et une autre branche pour la deuxième séance, sans avoir à réutiliser une branche déjà utilisée.

Cependant, il existe un risque que l'opérateur qui, après une première séance de dialyse, a déconnecté la source d'alimentation en dialysat par rapport à la première branche, connecte par mégarde la nouvelle source d'alimentation en dialysat sur la première branche déjà utilisée au lieu de la deuxième branche non encore utilisée. En outre, la deuxième branche non encore utilisée est censée être fermée par un bouchon pendant la première séance, mais il existe un risque que ce bouchon ait été enlevé par mégarde et que cette deuxième branche, qui pend alors sans être fermée, soit contaminée.

Il est ainsi souhaitable de pouvoir encore réduire le risque de contamination dans la machine de dialyse au cours d'opérations de l'utilisateur ou d'un tiers sur la machine, notamment lors du changement de la source d'alimentation en dialysat entre deux séances de dialyse.

La présente invention a pour but de proposer une nouvelle machine de dialyse, et un nouveau procédé correspondant permettant de pallier tout ou partie des problèmes exposés ci-dessus.

### RESUME DE L'INVENTION

A cet effet, l'invention a pour objet une machine de dialyse permettant de traiter un liquide corporel, tel que du sang ou du plasma, la machine de dialyse comportant :
- un dialyseur comprenant une enceinte qui inclut une zone de passage du liquide corporel qui présente une entrée de liquide corporel et une sortie de liquide corporel, et une zone de passage de dialysat qui présente une entrée de dialysat et une sortie de dialysat, et un système de membrane entre la zone de passage de liquide corporel et la zone de passage de dialysat ;
- un système de délivrance de dialysat,
- un système de raccordement permettant de raccorder une source d'alimentation en dialysat au système de délivrance de dialysat, le système de raccordement comprenant :
- un premier tronçon, qui comporte un conduit muni à une extrémité d'un premier dispositif de raccordement qui est connectable à un connecteur d'une première source d'alimentation en dialysat, et qui est déconnectable dudit connecteur de la première source d'alimentation en dialysat et muni à l'autre extrémité d'un deuxième dispositif de raccordement, ledit conduit étant un conduit souple de section constante à l'état non écrasé dudit conduit, et
- un deuxième tronçon qui comporte un conduit qui présente une extrémité raccordée à un système de délivrance de dialysat et qui est muni à une extrémité opposée d'un dispositif de raccordement connecté, de manière déconnectable, avec le deuxième dispositif de raccordement du premier tronçon, pour fournir une connexion en série du premier tronçon et du deuxième tronçon, tout en permettant de déconnecter ledit premier tronçon du deuxième tronçon, ledit conduit du deuxième tronçon étant un conduit souple de section constante à l'état non écrasé dudit conduit,
   de sorte que, à l'état déconnecté et retiré dudit premier tronçon par rapport au deuxième tronçon, ledit dispositif de raccordement du deuxième tronçon est raccordable à un connecteur d'une deuxième source d'alimentation en dialysat et déconnectable dudit connecteur de la deuxième source d'alimentation en dialysat.

Ainsi, le ou chaque autre tronçon qui est susceptible d'être utilisé ultérieurement pour une autre séance de dialyse, n'est pas laissé libre en attente d'utilisation comme dans la solution en Y de l'état de la technique, mais ledit ou chaque tronçon reste raccordé en série au tronçon qui est connecté à la source d'alimentation en dialysat pour permettre une communication fluidique entre la source d'alimentation et le système de délivrance de dialysat qui passe par ledit ou chacun desdits autres tronçons.

Le deuxième tronçon est initialement disposé entre le premier tronçon et le système de délivrance de dialysat.

La conception du système de raccordement sous forme de deux tronçons (ou plus lorsque le deuxième tronçon est formé de plusieurs tronçons connectés en série) qui sont connectés en série l'un à l'autre, permet de limiter le risque de contamination puisque le deuxième tronçon ne présente pas d'extrémité laissée libre qui pourrait trainer et être contaminée. En effet les extrémités de chaque tronçon du système de raccordement sont chacune raccordée soit à un autre tronçon, soit à la source d'alimentation (pour le tronçon le plus proche de la source d'alimentation) soit au système de délivrance (pour le tronçon le plus proche du système de délivrance).

En particulier dans le cas de deux tronçons, les extrémités du deuxième tronçon sont raccordées au premier tronçon et au système de délivrance. Le risque, comme dans l'état de la technique avec un système de raccordement en Y, que, après une première séance de dialyse, l'utilisateur branche la source d'alimentation sur le premier tronçon déjà utilisé est limité puisque ce premier tronçon est prévu pour être retiré.

En outre l'opérateur comprend et identifie visuellement que pour une séance de dialyse suivante, le système de raccordement doit être plus court que pour la séance de dialyse précédemment effectuée, du fait que le tronçon précédemment utilisé est prévu pour être retiré afin de pouvoir connecter la nouvelle source d'alimentation sur le prochain tronçon. Ainsi, le risque d'oubli de retirer le tronçon utilisé lors de la précédente séance de dialyse et de brancher dessus la nouvelle source d'alimentation en dialysat pour la séance de dialyse suivante est réduit.

La machine peut aussi comporter une ou plusieurs des caractéristiques suivantes prises dans toute combinaison techniquement admissible.

Selon un mode de réalisation, le système d'amenée du dialysat comprend un système de délivrance de dialysat raccordé à la ligne d'amenée, ledit système de délivrance comprenant au moins une poche souple, appelée poche ventricule, destinée à contenir du dialysat, et des moyens de mise sous pression de la poche ventricule.

Selon une caractéristique optionnelle, le dispositif comprend un élément de fermeture permettant, à l'état fermé, d'empêcher la circulation de liquide à l'intérieur du conduit du premier tronçon.

Selon une caractéristique optionnelle, l'élément de fermeture est de type inviolable de sorte que, à l'état fermé, l'élément de fermeture est verrouillé et/ou dégrade le conduit du premier tronçon pour empêcher la réutilisation du conduit après que l'élément de fermeture a été fermé.

Selon une caractéristique optionnelle, le deuxième tronçon comprend un élément d'ouverture et de fermeture activable et désactivable permettant d'autoriser ou d'empêcher la circulation de liquide à travers le conduit du deuxième tronçon.

Selon une caractéristique optionnelle, le premier dispositif de raccordement du premier tronçon comprend :
- un élément tubulaire apte à être engagé dans une ouverture du connecteur correspondant de la première source d'alimentation en dialysat pour permettre une communication de liquide à l'intérieur dudit élément tubulaire ; et
- une paroi périphérique qui s'étend autour et à écartement de l'élément tubulaire pour définir un espace annulaire d'insertion d'une paroi périphérique dudit connecteur de la première source d'alimentation en dialysat.

Selon un aspect particulier, la première source d'alimentation comprenant une poche, une conduite et ledit connecteur avec lequel le premier dispositif de raccordement du premier tronçon est apte à être connecté et déconnecté, est soudé à la conduite de la première source d'alimentation qui relie le connecteur à la poche de la première source d'alimentation. Autrement dit, le connecteur de la première source d'alimentation n'est pas démontable par rapport à la conduite ou à la poche de la première source d'alimentation.

Selon une caractéristique optionnelle, le dispositif de raccordement du deuxième tronçon comprend :
- un élément tubulaire apte à être engagé dans une ouverture du deuxième dispositif de raccordement du premier tronçon pour permettre une communication de liquide à l'intérieur dudit élément tubulaire, et qui est, à l'état retiré du premier tronçon, apte à être engagé dans une ouverture du connecteur de la deuxième source d'alimentation en dialysat pour permettre une communication de liquide à l'intérieur dudit élément tubulaire; et
- une paroi périphérique qui s'étend autour et à écartement de l'élément tubulaire pour définir un espace annulaire d'insertion d'une paroi périphérique dudit deuxième dispositif de raccordement du premier tronçon ou, à l 'état retiré du premier tronçon, pour définir un espace annulaire d'insertion d'une paroi périphérique du connecteur de la deuxième source d'alimentation en dialysat.

Selon une caractéristique optionnelle, le deuxième dispositif de raccordement du premier tronçon comprend une paroi périphérique munie d'un filetage apte à coopérer avec un taraudage ménagé dans la paroi périphérique du dispositif de raccordement du deuxième tronçon.

Selon une caractéristique optionnelle, la machine comprend :
- une ligne d'amenée de dialysat qui est raccordable à une entrée d'une zone de passage de dialysat du dialyseur, et
- une ligne d'évacuation de dialysat qui est raccordable à une sortie de la zone de passage de dialysat du dialyseur.

Selon une caractéristique optionnelle, la ligne d'évacuation comprend une conduite pour le raccordement à un dispositif d'évacuation tel qu'un système de mise à l'égout et/ou un système de récupération du dialysat usagé.

Selon une caractéristique optionnelle, la machine comprend au moins une poche souple, appelée poche ventricule, destinée à contenir du dialysat, formant partie dudit système de délivrance.

Selon une caractéristique optionnelle, ladite au moins une poche est inclue dans un dispositif, appelé cassette, qui est insérable et retirable d'un logement correspondant ménagé dans la machine de dialyse.

Selon une caractéristique optionnelle, la machine comprend un châssis et ledit dispositif, appelé cassette, est amovible par rapport au châssis de la machine.

L'invention concerne également un procédé pour raccorder successivement des sources d'alimentation en dialysat à un système de raccordement d'une machine selon l'un quelconque des modes de réalisation proposés ci-dessus, le procédé comprenant les étapes suivantes :
- fournir le système de raccordement dans une configuration où le premier tronçon est raccordé au deuxième tronçon , le deuxième tronçon étant raccordé par son autre extrémité au système de délivrance de dialysat ;
- fournir une première source d'alimentation en dialysat présentant un connecteur non raccordé au système de raccordement ;
- lorsqu'un bouchon ferme l'extrémité du premier tronçon opposée à celle raccordée au deuxième tronçon , retirer ledit bouchon ;
- raccorder le premier dispositif de raccordement du premier tronçon à la première source d'alimentation en dialysat , et après usage de la première source d'alimentation en dialysat :
- de préférence, obturer le premier tronçon , par exemple par fermeture d'un système de pince sur le premier tronçon,
- de préférence, retirer la première source d'alimentation en dialysat et fermer le dispositif de raccordement du premier tronçon à l'aide d'un bouchon ;
- retirer le premier tronçon par rapport au deuxième tronçon du dispositif de sorte que le deuxième tronçon présente un dispositif de raccordement libre;
- fournir une deuxième source d'alimentation en dialysat présentant un connecteur non raccordé au système de raccordement ;
- raccorder le connecteur de la deuxième source d'alimentation en dialysat au dispositif de raccordement libre dudit deuxième tronçon .

### BREVE DESCRIPTION DES DESSINS

D'autres caractéristiques et avantages de l'invention ressortiront encore de la description qui suit, laquelle est purement illustrative et non limitative et doit être lue en regard des dessins annexés, sur lesquels :

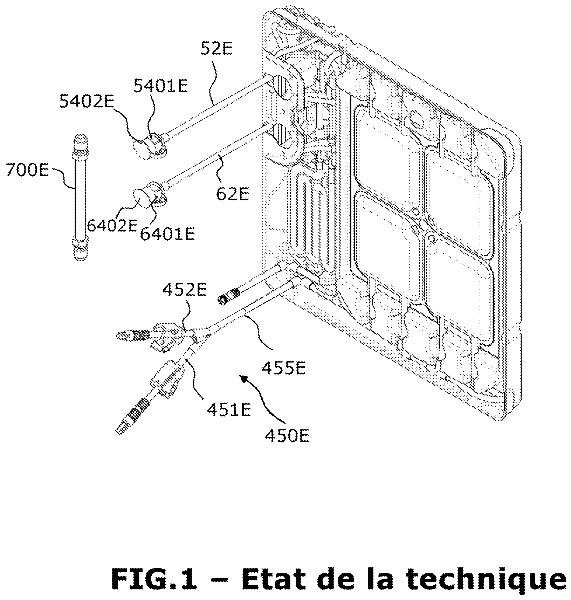

- la Figure 1 est une vue d'une partie d'une machine de dialyse selon un mode de réalisation connu de l'état de la technique ;
- la Figure 2 est une vue schématique d'une machine de dialyse conforme à un mode de réalisation de l'invention, configurée pour une première séance de dialyse ;
- la Figure 3 est une vue schématique d'une partie de la machine de dialyse de la Figure 2, au cours du remplacement de dispositifs de raccordement du dialyseur par de nouveaux dispositifs de raccordement destinés à être raccordés à des dispositifs de raccordements solidaires des lignes d'amenée et d'évacuation de dialysat, et à l'état retiré de la source d'alimentation en dialysat précédemment utilisée et du tronçon correspondant du système de raccordement au système de délivrance de dialysat;
- la Figure 4 est une vue schématique d'une machine de dialyse reprenant des éléments de la machine de dialyse des Figures 2 et 3 conformément à un mode de réalisation de l'invention, la machine étant configurée pour une deuxième séance de dialyse, le dialyseur ayant été remplacé par un nouveau dialyseur connecté aux lignes d'amenée et d'évacuation de dialysat à l'aide de nouveaux dispositifs de raccordement, et une nouvelle source d'alimentation en dialysat étant connectée à un tronçon restant du système de raccordement au système de délivrance;
- la Figure 5 est une vue schématique d'un dialyseur d'une machine de dialyse selon un mode de réalisation de l'invention, au cours d'une étape d'ultrafiltration ;
- la Figure 6 est une vue schématique d'un dialyseur d'une machine de dialyse selon un mode de réalisation de l'invention, au cours d'une étape de rétrofiltration ;
- la Figure 7 est une vue en perspective d'une partie de la machine de dialyse de la Figure 2, selon un mode de réalisation de l'invention, appelée cassette, montrant chaque système de raccordement au dialyseur des lignes d'amenée et d'évacuation de dialysat, à l'état couplé du deuxième dispositif de raccordement au premier dispositif de raccordement et à l'état fermé du bouchon du deuxième dispositif de raccordement, et montrant un système de raccordement pour raccorder une source d'alimentation en dialysat à un système de délivrance de dialysat pour une première séance de dialyse;
- la Figure 8 est une vue de chaque système de raccordement de dialyseur de la Figure 7 à l'état ouvert du bouchon du deuxième connecteur du deuxième dispositif de raccordement de chaque système de raccordement ;
- la Figure 9 est une vue du système de raccordement de la source d'alimentation en dialysat configuré pour une première séance de dialyse ;
- la Figure 10 est une vue de chaque système de raccordement de dialyseur de la Figure 8 à l'état déconnecté du deuxième dispositif de raccordement par rapport au premier dispositif de raccordement de chaque système de raccordement ;
- la Figure 11 est une vue du système de raccordement de source d'alimentation en dialysat, à l'état déconnecté du premier tronçon du système pour permettre de connecter une nouvelle source d'alimentation en dialysat à l'extrémité libre du deuxième tronçon du système pour une deuxième séance de dialyse ;
- la Figure 12 est une vue reprenant le premier dispositif de raccordement de chaque système de raccordement de la Figure 8 qui reste fixé à la ligne d'amenée ou d'évacuation correspondante, ainsi qu'un nouveau deuxième dispositif de raccordement, identique au deuxième dispositif de raccordement qui a été retiré, et dont le premier connecteur, qui est connectable au premier dispositif de raccordement, est muni d'un bouchon qui est représenté à l'état ouvert ;
- la Figure 13 est une vue des systèmes de raccordement au dialyseur de la Figure 12, à l'état connecté, pour chaque système de raccordement, du premier dispositif de raccordement avec le nouveau deuxième dispositif de raccordement, et à l'état ouvert du bouchon du deuxième connecteur du deuxième dispositif de raccordement de chaque système de raccordement ;
- la Figure 14 est une vue du système de raccordement de source d'alimentation en dialysat, à l'état connecté d'une nouvelle source d'alimentation en dialysat au deuxième tronçon restant du système de raccordement pour une deuxième séance de dialyse ;
- la Figure 15 est une vue en perspective d'une source d'alimentation en dialysat logée dans une structure support et connectable à la cassette de la machine de dialyse conformément à un mode de réalisation de l'invention ;

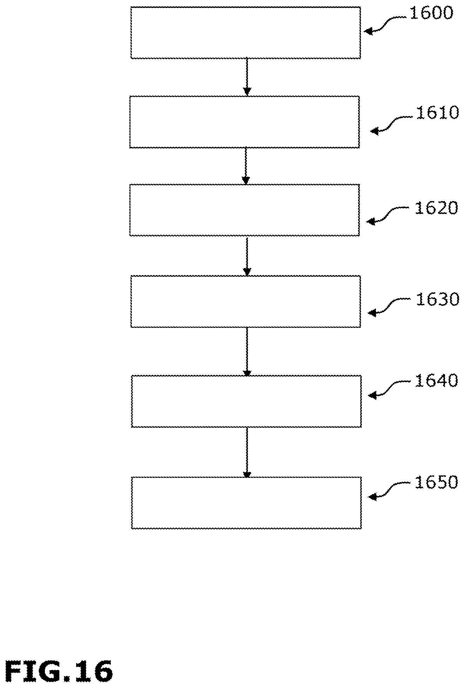

- la Figure 16 est un diagramme présentant plusieurs étapes d'un procédé de remplacement d'un dialyseur et d'une source d'alimentation en dialysat selon un mode de réalisation de l'invention.

### DESCRIPTION DETAILLEE

Le concept de l'invention est décrit plus complètement ci-après avec référence aux dessins joints, sur lesquels des modes de réalisation du concept de l'invention sont montrés. Sur les dessins, la taille et les tailles relatives des éléments peuvent être exagérées à des fins de clarté. Des numéros similaires font référence à des éléments similaires sur tous les dessins. Cependant, ce concept de l'invention peut être mis en œuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation.

### Aspects généraux

Comme rappelé ci-dessus, l'invention concerne un dispositif pour machine de dialyse, une machine de dialyse correspondante et un procédé d'utilisation correspondant permettant de traiter un liquide corporel, tel que du sang ou du plasma, avec un risque réduit de contamination lorsqu'un opérateur, qui peut être l'utilisateur ou un tiers, doit intervenir sur la machine, en particulier lors du changement du dialyseur (présenté ci-après) entre deux séances de dialyse, et/ou lors du changement de la source d'alimentation en dialysat (présentée ci-après) entre deux séances de dialyse.

Par liquide corporel, on entend un liquide du type de ceux présents dans le corps humain ou animal, tel que du sang ou du plasma.

La machine de dialyse permet de traiter un liquide corporel, tel que du sang ou du plasma. Par liquide corporel, on entend un liquide du type de ceux présents dans le corps humain ou animal, tel que du sang ou du plasma. Dans la suite de la description, le liquide corporel considéré est du sang mais bien entendu pourrait être un autre liquide corporel tel que du plasma.

Selon un mode de réalisation, la machine de dialyse peut reprendre les éléments décrits dans la demande internationale numéro WO2013050689A1 ou WO2019150058A2. La machine de dialyse selon l'invention se distingue cependant de celles décrites dans lesdites demandes internationale WO2013050689A1 et WO2019150058A2 au moins par le système de raccordement de la source d'alimentation en dialysat au système de délivrance du dialysat comme expliqué ci-après, et de préférence aussi, par le système de raccordement de la ligne d'amenée de dialysat à l'entrée du dialyseur et/ou par le système de raccordement de la ligne d'évacuation de dialysat à la sortie du dialyseur, comme expliqué ci-après.

### Dialyseur

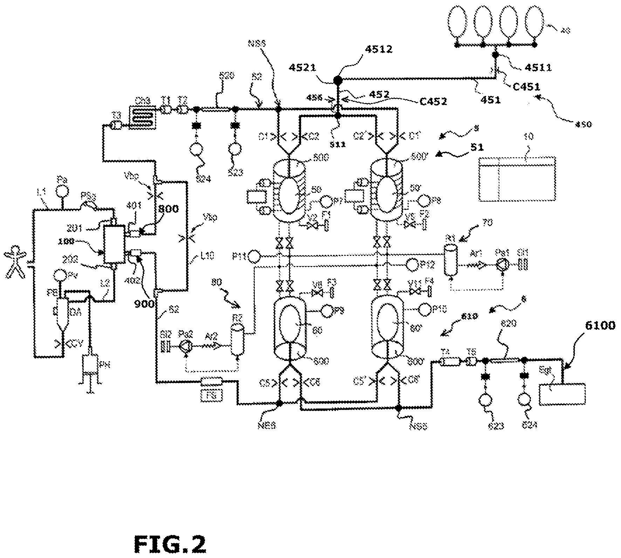

Ladite machine comporte un dialyseur 100. Le dialyseur est encore usuellement appelé filtre dialyseur. Comme illustré aux Figures 2, 5 et 6, le dialyseur 100 se présente sous la forme d'une enceinte qui inclut une zone de passage du liquide corporel 12, appelée compartiment sang, qui présente une entrée 201 de liquide corporel et une sortie 202 de liquide corporel. L'enceinte inclut aussi une zone de passage de dialysat 14, appelé aussi compartiment dialysat, qui présente une entrée de dialysat 401 et une sortie de dialysat 402.

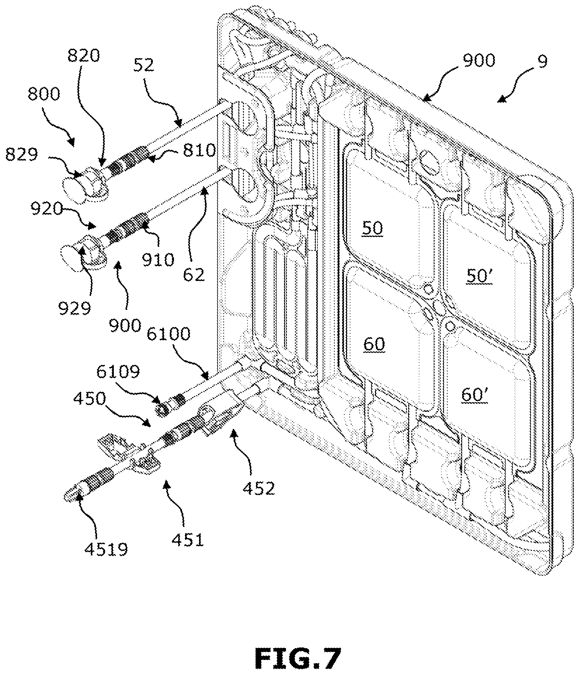

L'entrée de dialysat 401 et la sortie de dialysat 402 sont destinées à être raccordées par des systèmes de raccordement 800, 900 présentés ci-après à, respectivement, une ligne d'amenée 52 de dialysat et une ligne d'évacuation 62 de dialysat (voir Figure 7).

Le dialysat est un liquide bien connu de l'homme du métier dont la composition est proche du sérum physiologique. Le dialysat comprend de l'eau d'une qualité suffisante sur le plan minéral et sur le plan bactériologique. Cette eau est additionnée à un concentré, sous forme de poudre de sels minéraux ou sous forme de concentré liquide, pour fabriquer le dialysat requis.

Usuellement le sérum physiologique est composé d'un mélange d'eau et de chlorure de sodium. Le dialysat est généralement composé de sérum physiologique auquel sont ajoutés du glucose et des ions tels que potassium, magnésium calcium et éventuellement lactate et/ou bicarbonate selon les besoins du patient.

Selon un aspect préférentiel, le dialysat utilisé est du dialysat stérile apyrogène. Le dialysat a été stérilisé par exemple dans un autoclave. Ce dialysat peut être réalisé à partir d'eau purifiée et de sel.

La circulation de dialysat passe par le compartiment du dialysat 14, généralement à contre-sens de la circulation sanguine dans l'autre compartiment sang 12. Le dialysat circule généralement en circuit ouvert. On peut aussi prévoir une boucle de régénération partielle de dialysat propre à partir du dialysat chargé des toxines.

### Membrane

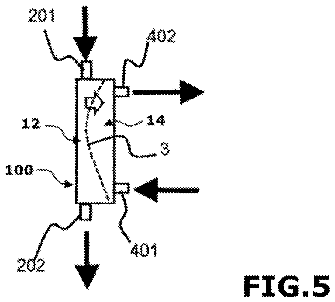

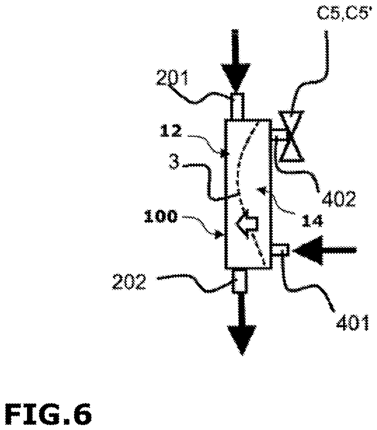

Comme illustré aux Figures 5 et 6, l'enceinte inclut aussi un système de membrane 3 pour permettre un échange d'éléments (substances, molécules) entre le liquide corporel présent dans la zone de passage de liquide corporel 12 et le dialysat présent dans la zone de passage de dialysat 14.

D'un point de vue fonctionnel, le dialyseur 100 peut être schématisé sous la forme d'une enceinte logeant une membrane 3 de dialyse qui sépare l'enceinte en un compartiment sang, correspondant à la zone de passage du liquide corporel 12 et en un compartiment dialysat, correspondant à la zone de passage de dialysat 14.

Le système de membrane 3 de dialyse, semi-perméable, est conçu de manière à laisser passer une fraction du volume sanguin lorsque la différence entre la pression locale dans le compartiment sang 12 et la pression locale dans le compartiment dialysat 14 est supérieure à une valeur donnée. Cette différence de pression est appelée pression transmembranaire. En particulier, le système de membrane est conçu de telle sorte que seules les molécules les plus petites peuvent passer à travers les trous ou pores du système de membrane, à savoir l'eau, les sels minéraux et les molécules de petit à moyen poids moléculaire.

Plus précisément, le système de membrane 3 peut comprendre une membrane conçue de telle sorte que, lorsque la différence entre la pression qui s'exerce sur au moins une portion de la membrane du côté du compartiment sang 12 et la pression qui s'exerce sur ladite portion de la membrane du côté du compartiment de dialysat 14, est supérieure à une valeur seuil donnée, ladite portion de la membrane laisse passer une fraction aqueuse du sang dans le compartiment de dialysat 14, selon un phénomène convectif appelé ultrafiltration. Inversement, ladite membrane 3 est conçue de telle sorte que, lorsque la différence entre la pression, qui s'exerce sur ladite portion de la membrane 3 du côté du compartiment de dialysat 14, et la pression, qui s'exerce sur ladite portion de la membrane du côté du compartiment sang 12, est supérieure à une valeur seuil donnée, ladite portion de la membrane laisse passer le dialysat dans le compartiment sang 12, selon un phénomène appelé rétrofiltration.

Le système de membrane de dialyse peut être réalisé sous forme de fibres capillaires, le sang circulant à l'intérieur des fibres, le dialysat à l'extérieur.

L'entrée 201 de sang est raccordée à une ligne artérielle L1 dont une extrémité est, pour procéder à une séance de dialyse, raccordée à une fistule du patient, afin de pouvoir extraire le sang du corps du patient à traiter. En outre, la sortie 202 de sang est raccordée à une ligne veineuse L2, dont une extrémité est raccordée à une veine du patient pour réintroduire le sang dans le corps du patient après traitement. On entend par "ligne" une tubulure comprenant éventuellement plusieurs branches ou portions et à travers laquelle un liquide peut circuler. Selon un aspect particulier ces lignes peuvent être liées l'une à l'autre de sorte que l'on ne peut retirer l'une sans l'autre.

Dans l'exemple illustré à la Figure 2, la ligne artérielle L1 est aussi munie d'une pompe à sang Psg, et d'un capteur de pression Pa, encore appelé capteur de pression artérielle. La ligne veineuse L2 comprend un capteur de pression Pv, encore appelé capteur de pression veineuse, un piège à bulle PB, et un détecteur d'air DA. Avantageusement, le capteur de pression Pv est connecté sur le piège à bulle PB. Préférentiellement, ladite ligne veineuse L2 comprend aussi un système d'injection d'anticoagulant, tel qu'une pompe à héparine PH.

La ligne artérielle L1, la ligne veineuse L2 et le compartiment sang 12 forment le circuit sanguin de la machine.

### Circuit de dialysat

Comme illustré à la Figure 2, la machine comprend aussi un système d'amenée du dialysat 5 qui comporte une ligne d'amenée 52 de dialysat qui est raccordée à l'entrée 401 du compartiment de dialysat 14, et un système d'évacuation du dialysat 6 qui comporte une ligne d'évacuation 62 raccordée à la sortie 402 du compartiment de dialysat 14.

Ledit système d'amenée du dialysat 5, le compartiment de dialysat 14, et ledit système d'évacuation du dialysat 6 forment le circuit de dialysat.

Comme rappelé ci-dessus, la ligne d'amenée 52 de dialysat est raccordée à l'entrée 401 de la zone de passage de dialysat 14 par un système de raccordement 800.

### Système de raccordement 800 de ligne d'amenée

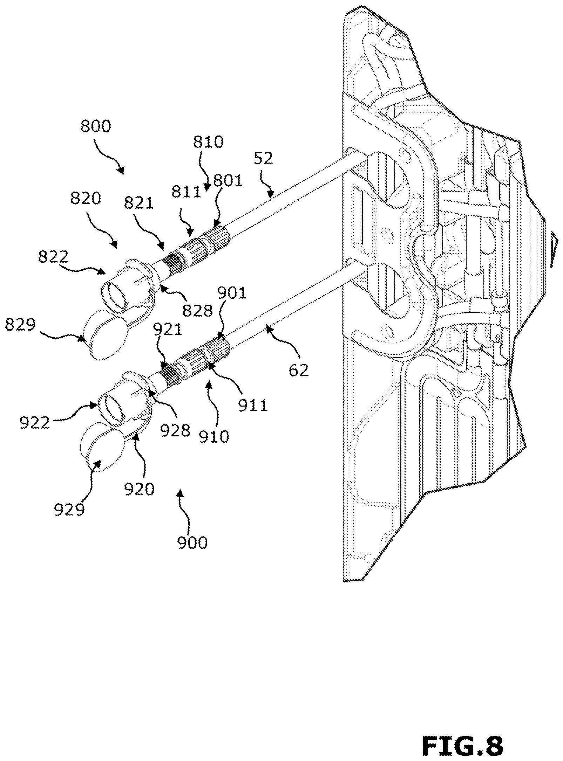

Comme illustré plus particulièrement à la Figure 8, le système de raccordement 800 comprend un premier dispositif de raccordement 810 fixé, par exemple par collage, à la ligne d'amenée 52, et un deuxième dispositif de raccordement 820 connectable au premier dispositif de raccordement 810, pour permettre une communication fluidique à travers le premier dispositif de raccordement et le deuxième dispositif de raccordement. Le deuxième dispositif de raccordement 820 est déconnectable par rapport au premier dispositif de raccordement 810.

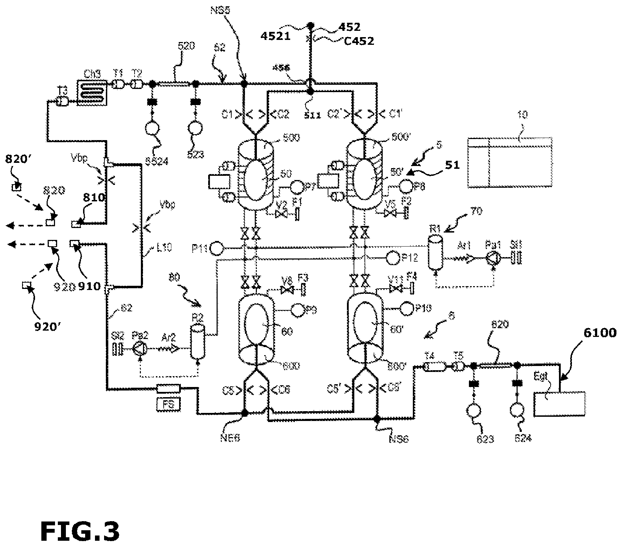

Le deuxième dispositif de raccordement 820, connecté au premier dispositif de raccordement 810, peut ainsi être connecté à l'entrée de dialysat 401 du dialyseur 100 pour une première séance de dialyse, puis être déconnecté du premier dispositif de raccordement 810 qui reste fixé sur la ligne d'amenée, par exemple en même temps que le dialyseur est retiré (ou séparément), pour pouvoir remplacer ce deuxième dispositif de raccordement 820, par un nouveau deuxième dispositif de raccordement 820' comme illustré aux Figures 3, 4, 12 et 13.

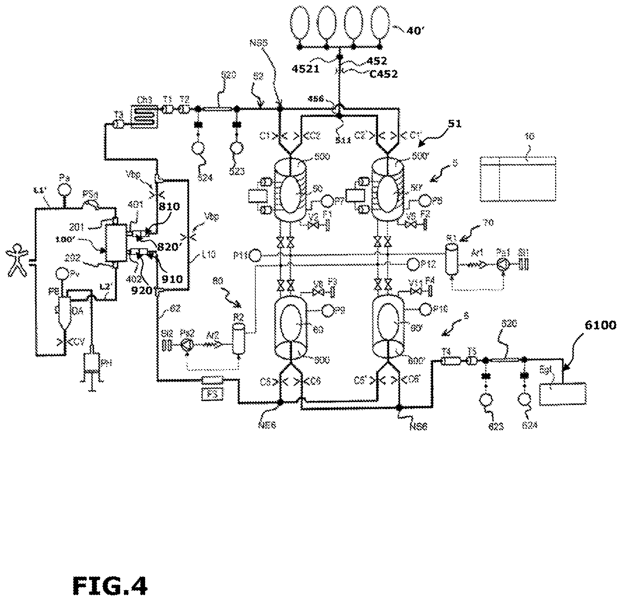

Ce nouveau deuxième dispositif de raccordement 820', considéré comme propre ou stérile, peut alors être connecté par une extrémité au premier dispositif de raccordement 810 et par son autre extrémité à l'entrée de dialysat 401 d'un nouveau dialyseur 100' comme illustré à la Figure 4.

Dans le mode de réalisation illustré à la Figure 8, le deuxième dispositif de raccordement 820, qui est connectable de manière amovible au premier dispositif de raccordement 810, comprend un connecteur 822 de type Hansen femelle et un connecteur 821 de type Luer Lock femelle. Ces deux connecteurs 821, 822 sont reliés entre eux par une tubulure. Selon un aspect particulier, les raccords de type Hansen correspondent à la norme NF EN ISO 8637. Préférentiellement, le premier dispositif de raccordement 810 fixé à la ligne d'amenée 52 comprend un raccord 811 de type Lueur Lock mâle raccordable au connecteur 821, et un raccord 801 de type Lueur Lock femelle au niveau de l'autre côté raccordé à la ligne 52. Selon un aspect particulier, les raccords de type Lueur Lock correspondent à la norme NF EN ISO 594.

Le connecteur 811 comprend un élément tubulaire 812 apte à être engagé dans l'ouverture du premier connecteur 821 du deuxième dispositif de raccordement 820, pour être en communication fluidique avec l'ouverture du premier connecteur 821 du deuxième dispositif de raccordement 820, et une paroi périphérique 813 qui s'étend autour et à écartement de l'élément tubulaire 812 pour définir un espace annulaire d'insertion de la paroi périphérique du premier connecteur 821 du deuxième dispositif de raccordement 820 présenté ci-après. Avantageusement, la paroi périphérique 813 est taraudée pour coopérer avec un filetage 821a du premier connecteur 821 ménagé sur au moins une partie de la face externe de sa paroi périphérique.

Le premier connecteur 821 du deuxième dispositif de raccordement 820 se présente sous la forme d'un élément tubulaire, de préférence droit, qui définit une ouverture débouchant à une extrémité dudit deuxième dispositif de raccordement 820 pour recevoir ledit élément tubulaire 812 du connecteur 811. Le premier connecteur 821 est ainsi apte à coopérer avec le connecteur 811 du premier dispositif de raccordement 810 par engagement de la paroi périphérique du premier connecteur 821 entre l'élément tubulaire 812 et la paroi périphérique 813 du connecteur 811 du premier dispositif de raccordement 810.

Le deuxième connecteur 822 du deuxième dispositif de raccordement 820, opposé au premier connecteur 821, peut se présenter sous la forme d'un élément tubulaire de section circulaire, de préférence de diamètre supérieur à celui du premier connecteur 821. Le deuxième connecteur 822 est connectable à l'entrée de dialysat 401 du dialyseur.

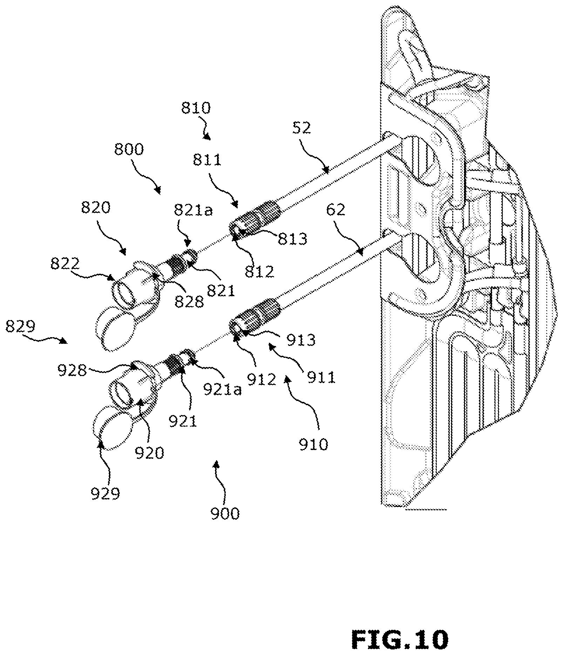

Selon un mode de réalisation et comme illustré à la Figure 10, le deuxième dispositif de raccordement 820, présente des ailettes 828 latérales facilitant la saisie du deuxième dispositif de raccordement 820 pour le déconnecter.

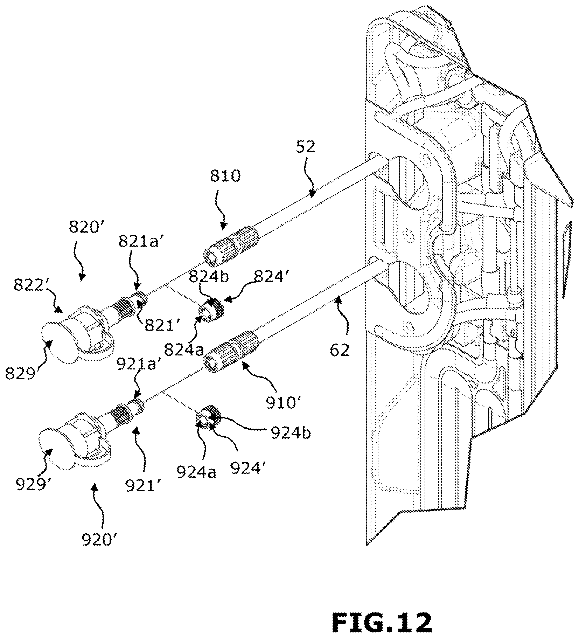

A l'état non encore connecté du deuxième dispositif de raccordement 820 au premier dispositif de raccordement 810, le deuxième dispositif de raccordement 820 est muni d'un bouchon qui permet de fermer l'ouverture du premier connecteur 821 du deuxième dispositif de raccordement 820, tout en étant retirable pour libérer l'ouverture du premier connecteur 821 du deuxième dispositif de raccordement 820 et pouvoir connecter le deuxième dispositif de raccordement 820 au premier dispositif de raccordement 810. Un tel bouchon est visible et référencé 824' à la Figure 12 pour le deuxième dispositif de raccordement 820'.

Le bouchon 824' comprend un plot 824a apte à être engagé dans l'ouverture du premier connecteur 821' du deuxième dispositif de raccordement 820', et une paroi périphérique 824b qui s'étend autour et à écartement du plot 824a pour définir un espace annulaire d'insertion de la paroi périphérique du premier connecteur 821'. La paroi périphérique 824b est de préférence taraudée pour coopérer avec le filetage 821a'.

Le deuxième connecteur 822 du deuxième dispositif de raccordement 820, destiné à être connecté à l'entrée 401 du dialyseur 100, est muni d'un bouchon 829 permettant de fermer l'ouverture du deuxième connecteur 822, tout en étant retirable pour libérer l'ouverture du deuxième connecteur 822. Avantageusement, le bouchon 829 reste attaché au deuxième connecteur 822 par un élément d'attache tel qu'un fil, pour éviter qu'il ne tombe ou ne soit perdu.

Comme illustré aux Figures 3, 4, 12 et 13, le deuxième dispositif de raccordement 820 peut être retiré et remplacé par un nouveau deuxième dispositif de raccordement 820', identique à celui retiré, ce qui permet de limiter le risque de contamination lors du changement du dialyseur au niveau de l'extrémité de la ligne d'amenée destinée à être raccordée au dialyseur, tout en n'ayant à changer qu'une partie du système de raccordement qui est susceptible d'avoir été contaminée au niveau du bouchon resté ouvert En particulier un tel système permet de limiter le risque de contamination/prolifération bactériologique, pendant une période d'arrêt d'utilisation de la machine (ou mise en veille) entre une première séance et une deuxième séance de dialyse, puisque le nouveau deuxième dispositif de raccordement 820' peut être raccordé et laissé en position de fermeture du bouchon 829 pendant la période d'arrêt d'utilisation de la machine (ou mise en veille) entre la première séance et la deuxième séance de dialyse.

La réalisation du système de raccordement 800 en deux parties (de même que pour le système 900 présenté ci-après) avec une partie fixe qui reste sur la ligne d'amenée, et une partie connectable au dialyseur qui est déconnectable de la partie fixe, permet de remplacer ladite partie connectable au dialyseur qui est susceptible d'être contaminée, par une nouvelle partie identique stérile, et ainsi de limiter le risque de contamination lors du remplacement du dialyseur.

### Système de délivrance 51 de dialysat

Le système d'amenée 5 comprend aussi un système de délivrance 51 de dialysat raccordé à la ligne d'amenée 52. Ledit système d'amenée du dialysat 5 permet de délivrer du dialysat dans le compartiment dialysat 14 par la ligne d'amenée 52.

Selon un mode de réalisation, ledit système de délivrance 51 comprend au moins une poche 50 souple, appelée poche ventricule, destinée à contenir du dialysat. Ledit système 51 peut comprendre plusieurs poches comme dans l'exemple illustré aux figures.

Préférentiellement, ledit système d'amenée du dialysat 5 comprend une poche ventricule supplémentaire 50' qui est montée en dérivation de la portion de la ligne d'amenée 52 sur laquelle est raccordée ladite poche ventricule 50.

Dans la suite de la description, il est fait référence à un système 51 comprenant plusieurs poches 50, 50', mais on comprend que cette description s'applique aussi au cas d'un système de délivrance 51 comprenant une seule poche. Chaque poche ventricule du système 51 est destinée à contenir du dialysat et est raccordée à ladite ligne d'amenée 52.

Chaque poche ventricule 50, 50' loge dans une enceinte 500, 500' sensiblement étanche qui peut être mise sous pression, et éventuellement en dépression. Chaque poche ventricule 50, 50' peut ainsi être mise sous pression par des moyens de mise sous pression 70 et, éventuellement, en dépression par un générateur de vide 80.

Lesdits moyens de mise sous pression 70 comprennent un dispositif d'injection de gaz sous pression, tel qu'un compresseur d'air ou une bouteille de gaz comprimé, apte à injecter du gaz sous pression dans l'enceinte 500, 500' étanche dans laquelle loge la poche ventricule 50, 50'. Ladite enceinte présente une sortie dont l'ouverture est contrôlable par une électrovanne pour réduire la pression dans l'enceinte.

L'application d'une pression sur la poche ventricule 50 ou 50' et la régulation de cette pression appliquée, à l'aide de la différence de pression mesurée aux bornes du tube à perte de charge 520, permet de maintenir pendant un temps donné un débit fixe de dialysat dans le compartiment dialysat de manière fiable et précise, et ainsi de maîtriser des opérations de rétrofiltration. L'application et la régulation de la pression appliquée sur la poche ventricule 50 ou 50' permet aussi, en ajustant la pression appliquée à la poche de décharge 60 ou 60' (présentée ci-après), de maintenir pendant un temps donné une pression moyenne donnée dans le compartiment dialysat, et ainsi de maitriser des opérations d'ultrafiltration.

Les deux poches ventricules 50, 50' sont aptes à être mises sous pression/dépression indépendamment l'une de l'autre.

### Système d'ouverture/fermeture de la ligne d'amenée

Comme illustré par exemple à la Figure 2, chaque poche ventricule 50, 50' est aussi munie d'un organe aval de fermeture/ouverture C1, C1' de la ligne d'amenée qui, à l'état ouvert, autorise l'écoulement de dialysat depuis la poche 50, 50' correspondante vers l'entrée 401 du compartiment dialysat, sous l'effet d'une pression appliquée sur ladite poche ventricule par les moyens 70 correspondants, et à l'état fermé, empêche ledit écoulement de dialysat.

Chaque organe aval de fermeture/ouverture C1, C1' de la ligne d'amenée est situé entre la poche correspondante 50, 50' et le nœud de sortie NS5 de raccordement des deux poches 50, 50'. Chaque poche ventricule 50, 50' est aussi munie d'un organe amont de fermeture/ouverture C2, C2' qui permet d'autoriser ou non l'alimentation de ladite poche ventricule 50, 50' par une source d'alimentation 40. La source d'alimentation 40 peut comprendre une pluralité de poches nourricières.

Un système de raccordement 450 permettant de raccorder une source d'alimentation en dialysat au système délivrance 51 de dialysat est décrit par la suite.

Dans l'exemple illustré aux Figures 2 à 4, chaque portion de la ligne d'amenée 52 de dialysat au niveau de laquelle est situé un organe amont C2, C2' ou aval C1, C1' de fermeture/ouverture, est souple. Chacun desdits organes C2, C2', C1, C1' de fermeture/ouverture est formé par une pince commandable pour, d'une part, pincer de l'extérieur la paroi de ladite portion souple de la ligne d'amenée 52 de manière à fermer cette portion, et, d'autre part, pour laisser ouverte ladite portion souple de ladite ligne d'amenée 52.

La présence des deux poches ventricules et du système de pinçage alternatif formé par les organes C1 à C2' permet de charger successivement et alternativement les poches ventricules à partir de la source d'alimentation 40. Ceci permet d'alimenter en continu la ligne d'amenée du dialysat à partir de l'une des poches ventricules.

La mise en dépression de l'enceinte d'une poche ventricule 50 ou 50' à l'aide du générateur de vide 80 permet, à l'état ouvert de l'organe amont d'ouverture/fermeture C2 ou C2', et à l'état fermé de l'organe aval d'ouverture/fermeture correspondant C1 ou C1', d'aspirer le dialysat contenu dans la source d'alimentation 40 ou 40' pour remplir ladite poche ventricule 50 ou 50'.

Pour commander l'écoulement de dialysat dans la ligne d'amenée 52, l'unité de pilotage 10 commande l'ouverture de l'organe aval C1 ou C1' de l'une des poches ventricules 50 ou 50' et la fermeture de l'organe amont C2 ou C2' correspondant. L'utilisation de deux poches ventricules montées en parallèle permet d'en recharger une pendant que l'autre est en cours d'utilisation.

Selon un mode de réalisation, la ligne 52 d'amenée de dialysat comporte une restriction de passage 520 pour créer une perte de charge et permettre de réguler la pression appliquée à la poche ventricule 50 ou 50' en fonction de la différence de pression mesurée aux bornes de ladite restriction. En particulier, ladite ligne 52 est munie de moyens de mesure de la différence de pression entre l'entrée et la sortie de ladite restriction de passage 520. Cette restriction 520 peut être formée au moins en partie d'un tube calibré de section constante qui forme un étranglement sur une longueur prédéterminée. Ladite restriction de passage 520 est située entre les poches 50, 50' et l'entrée 401 du compartiment dialysat 14.

La connexion NS5 du système de délivrance 51 à ladite ligne d'amenée 52 est située en amont de la restriction de passage 520.

Lesdits moyens de mise sous pression maintiennent avantageusement une pression sur la poche ventricule 50 ou 50' qui est suffisante pour provoquer l'écoulement du dialysat. La ligne d'amenée est calibrée pour que, pour une pression déterminée appliquée à la poche ventricule 50 ou 50', le débit de dialysat dans ladite ligne ait une valeur sensiblement constante.

Un tel système formé d'un tube à perte de charge et de moyens de mesure de la différence de pression aux bornes de la restriction, permet notamment de déterminer le débit de dialysat qui circule dans ladite ligne d'amenée et qui entre dans le compartiment dialysat. Grâce au débit de dialysat ainsi mesuré, l'unité de pilotage 10 peut réguler la pression appliquée à la poche ventricule 50 ou 50' pour obtenir le débit de dialysat souhaité entrant dans le compartiment dialysat et maintenir précisément et de manière fiable ce débit à une valeur donnée pendant un temps donné.

Lesdits moyens de mesure de la différence de pression aux bornes de la restriction 520 de la ligne d'amenée 52 peuvent comprendre deux orifices de prise de pression ménagés, dans la paroi périphérique de ladite ligne 52, l'un en amont et l'autre en aval de ladite restriction 520. Chaque orifice de prise de pression est associé à un capteur de pression 523, 524. Chaque capteur de pression 523, 524 est agencé par rapport à l'orifice de prise de pression correspondant de manière à mesurer la pression dans la ligne d'amenée 52 au niveau dudit orifice de prise de pression tout en étant écarté dudit orifice pour ne pas être en contact avec le dialysat circulant dans la ligne 52, 62.

Les capteurs de pression 523, 524 peuvent être configurés comme décrits dans la demande internationale WO2013050689.

On peut prévoir que la mesure de pression dans la ligne artérielle L1 et la ligne veineuse L2 soit réalisée de la même manière que pour la prise de pression aux bornes de la restriction 520. On peut ainsi prévoir que le capteur de pression Pa et le capteur de pression Pv soient fixés dans une partie du bâti à écartement d'un orifice de pression ménagé dans la ligne L1, L2 correspondante. Selon un aspect particulier, les capteurs de pression Pa et Pv mesurent la pression dans la ligne L1, L2 correspondante sans risque de contamination.

### Système d'évacuation du dialysat

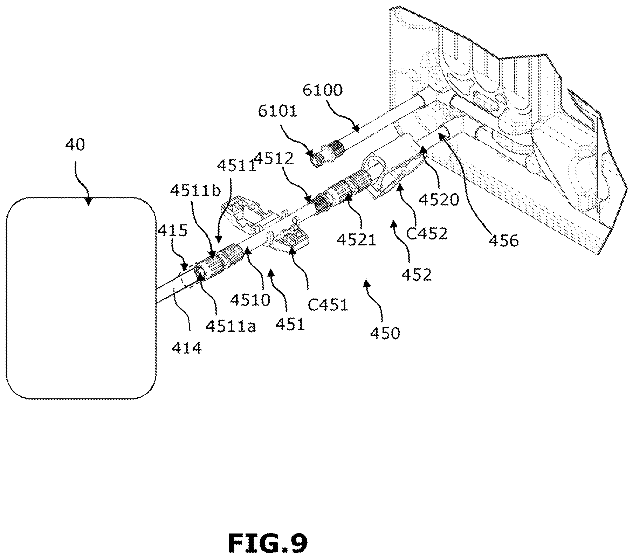

Le système d'évacuation du dialysat 6 comprend une ligne d'évacuation 62 de dialysat qui est raccordée à la sortie 402 du compartiment de dialysat 14 comme détaillé ci-après. Dans l'exemple illustré plus particulièrement aux Figures 7 et 9, le système d'évacuation du dialysat 6 comprend aussi une conduite 6100 destinée à être raccordée, par exemple au niveau de l'embout 6101 (qui peut être refermé par un bouchon 6109), à un récipient de récupération ou à un système de mise à l'égout Egt.

### Système de raccordement 900

Comme illustré plus particulièrement à la Figure 8 et de manière similaire au système de raccordement 800, le système de raccordement 900 comprend un premier dispositif de raccordement 910 fixé à la ligne d'évacuation 62, et un deuxième dispositif de raccordement 920 connectable au premier dispositif de raccordement 910, pour permettre une communication fluidique à travers le premier dispositif de raccordement et le deuxième dispositif de raccordement. Le deuxième dispositif de raccordement 920 est déconnectable par rapport au premier dispositif de raccordement 910.

Le deuxième dispositif de raccordement 920, connecté au premier dispositif de raccordement 910, peut ainsi être connecté à la sortie de dialysat 402 du dialyseur 100 pour une première séance de dialyse, puis être déconnecté du premier dispositif de raccordement 910, par exemple en même temps que le dialyseur est retiré (ou séparément), pour pouvoir remplacer ce deuxième dispositif de raccordement 920, par un nouveau deuxième dispositif de raccordement 920'.

Ce nouveau deuxième dispositif de raccordement 920', considéré comme propre ou stérile, peut alors être connecté par une extrémité au premier dispositif de raccordement 910 et par son autre extrémité à la sortie de dialysat 402 du nouveau dialyseur 100'.

Dans le mode de réalisation illustré à la Figure 8, le deuxième dispositif de raccordement 920, qui est connectable de manière amovible au premier dispositif de raccordement 910, comprend un connecteur 922 de type Hansen femelle et un connecteur 921 de type Luer Lock femelle. Ces deux connecteurs 921, 922 sont reliés entre eux par une tubulure. Selon un aspect particulier, les raccords de type Hansen correspondent à la norme NF EN ISO 8637. Préférentiellement, le premier dispositif de raccordement 910 fixé à la ligne d'évacuation 62 comprend un raccord 911 de type Lueur Lock mâle raccordable au connecteur 921, et un raccord 901 de type Lueur Lock femelle au niveau de l'autre côté raccordé à la ligne 52. Selon un aspect particulier, les raccords de type Lueur Lock correspondent à la norme NF EN ISO 594.

Dans l'exemple illustré aux Figures 2 à 15, le système de raccordement 900 comprend des éléments identiques à ceux du système de raccordement 800 présentés ci-dessus.

Le connecteur 911comprend un élément tubulaire 912 apte à être engagé dans l'ouverture du premier connecteur 921 du deuxième dispositif de raccordement 920, pour être en communication fluidique avec l'ouverture du premier connecteur 921 du deuxième dispositif de raccordement 920, et une paroi périphérique 913 qui s'étend autour et à écartement de l'élément tubulaire 912 pour définir un espace annulaire d'insertion de la paroi périphérique du premier connecteur 921 du deuxième dispositif de raccordement 920 présenté ci-après.

Préférentiellement et comme illustré à la Figure 10, ledit premier connecteur 921 est muni d'un filetage 921a, sur au moins une partie de la face externe de sa paroi périphérique apte à coopérer avec un taraudage ménagé à l'intérieur de la paroi 913.

Le premier connecteur 921 est apte à coopérer avec le connecteur 911 du premier dispositif de raccordement 910 par engagement de la paroi périphérique du premier connecteur 921 entre l'élément tubulaire 912 et la paroi périphérique 913 du connecteur 911.

Le deuxième connecteur 922 du deuxième dispositif de raccordement 920, opposé au premier connecteur 921, peut se présenter sous la forme d'un élément tubulaire de section circulaire, de préférence de diamètre supérieur à celui du premier connecteur 921. Le deuxième connecteur 922 est connectable à la sortie de dialysat 402 du dialyseur.

Selon un mode de réalisation et comme illustré à la Figure 10, le deuxième dispositif de raccordement 920, présente des ailettes 928 latérales facilitant la préhension du deuxième dispositif de raccordement 920 pour le retirer.

A l'état non encore connecté du deuxième dispositif de raccordement 920 au premier dispositif de raccordement 910, le deuxième dispositif de raccordement 920 comprend un bouchon qui permet de fermer l'ouverture du premier connecteur 921 du deuxième dispositif de raccordement 920, tout en étant retirable pour libérer l'ouverture du premier connecteur 921 du deuxième dispositif de raccordement 920. Ce bouchon est visible et référencé 924' à la Figure 12 pour le deuxième dispositif de raccordement 920'.

Le bouchon 924' comprend un plot 924a apte à être engagé dans l'ouverture du premier connecteur 921' du deuxième dispositif de raccordement 920', et une paroi périphérique 924b qui s'étend autour et à écartement du plot 924a pour définir un espace annulaire d'insertion de la paroi périphérique du premier connecteur 921', et de préférence taraudée pour coopérer avec le filetage 921a.

Le deuxième connecteur 922 du deuxième dispositif de raccordement 920 est muni du bouchon 929 permettant de fermer l'ouverture du deuxième connecteur 922, tout en étant retirable pour libérer l'ouverture du deuxième connecteur 922. Avantageusement, le bouchon 929 est attaché au deuxième connecteur 922 par un élément d'attache, tel qu'un fil, pour éviter qu'il ne tombe ou ne soit perdu.

Le bouchon 929' du deuxième dispositif de raccordement 920' peut être laissé fermé pendant la période d'arrêt ou mise en veille entre deux séances de dialyse, avant le raccordement du nouveau dialyseur 100'.

De manière similaire au système de raccordement 800, le système de raccordement 900 permet de limiter le risque de contamination/prolifération bactériologique au niveau de la ligne 62 correspondante, entre une première séance et une deuxième séance.

### Système d'ouverture/fermeture de la ligne d'évacuation

Ladite machine comprend un système, de préférence de type pince, pour l'ouverture/fermeture de la ligne 62 d'évacuation de dialysat. Le système d'ouverture/fermeture autorise, à l'état d'ouverture, la circulation dans ladite ligne d'évacuation 62 du dialysat présent en sortie 402 du compartiment de dialysat 14 pour remplir l'une des poches de décharge 60 ou 60', et empêche, à l'état de fermeture, la circulation dans ladite ligne d'évacuation 62 du dialysat de sorte que, pour une pression suffisante dans le compartiment dialysat, ledit dialysat passe à travers la membrane 3 dans le compartiment sang.

Dans l'exemple illustré à la Figure 2, ledit système d'évacuation du dialysat 6 comprend au moins une poche de décharge 60, de préférence souple, présentant une entrée et une sortie raccordées à la ligne d'évacuation de dialysat 62. Ledit système d'ouverture/fermeture comprend un organe amont d'ouverture/fermeture C5 situé entre la sortie 402 du compartiment dialysat 14 et ladite poche de décharge 60. Il peut aussi être prévu un organe de fermeture/ouverture sur la ligne 62.

Avantageusement, ledit système d'évacuation du dialysat 6 comprend au moins une autre poche de décharge 60' montée en dérivation de ladite poche de décharge 60. Ainsi la poche de décharge 60 et la poche de décharge 60' sont situées sur deux branches de la ligne d'évacuation 62. Autrement dit, en partant de la sortie du compartiment de dialysat 14, la ligne d'évacuation 62 se sépare, en amont des poches de décharge 60, 60', en deux branches parallèle munies l'une de la poche de décharge 60 et l'autre de la poche de décharge 60'. Ces deux branches se raccordent l'une à l'autre en aval des poches 60, 60' et en amont de la restriction de section 620 comme détaillé ci-après.

Ledit système d'ouverture/fermeture comprend un autre organe amont d'ouverture/fermeture C5' situé entre ladite autre poche de décharge 60' et le nœud d'entrée NE6 de raccordement des poches de décharge 60, 60'.

De manière similaire aux organes amont C5, C5', chaque poche de décharge 60, 60' est munie d'un organe aval C6, C6' de fermeture/ouverture situé entre la poche de décharge 60, 60' correspondante et le nœud de sortie NS6 de raccordement des poches de décharge 60, 60'.

Chaque portion de la ligne d'évacuation de dialysat 62 au niveau de laquelle est situé un organe amont C5, C5' ou aval C6, C6' de fermeture/ouverture est souple. Chacun desdits organes amont C5, C5' ou aval C6, C6' de fermeture/ouverture de la ligne d'évacuation de dialysat 62 est formé par une pince commandable pour, d'une part, pincer de l'extérieur la paroi de ladite portion souple de la ligne d'évacuation 62 de manière à fermer cette portion, et, d'autre part, pour laisser ouverte ladite portion souple de ladite ligne d'évacuation 62.

Un tel ensemble d'organes amont C5, C5' et aval C6, C6' de fermeture/ouverture associés aux poches de décharge 60, 60' permet à l'état ouvert de l'organe amont C5, respectivement C5', et fermé de l'organe aval C6, respectivement C6', de remplir la poche de décharge 60, respectivement 60', et, à l'état fermé de l'organe amont C5, respectivement C5', et ouvert de l'organe aval C6, respectivement C6', de vider ladite poche de décharge 60, respectivement 60'.

Dans le cas où le système d'évacuation 6 est formé par une ou plusieurs poches de décharge 60, 60', on considère que la ligne d'évacuation 62 est ouverte lorsque le dialysat peut circuler à travers ladite ligne pour remplir ladite ou l'une desdites poches.

Les moyens d'évacuation du dialysat et du liquide présents dans la poche de décharge 60 ou 60' peuvent être formés par la gravité si la configuration de la poche de décharge s'y prête et/ou par des moyens de mise sous pression 70, de préférence communs à ceux qui servent à la mise sous pression des poches ventricules 50, 50'. Dans ce dernier cas, ladite ou chaque poche 60, 60' est logée dans une enceinte 600, 600' sensiblement étanche apte à être mise sous pression par lesdits moyens de mise sous pression 70. Ladite enceinte présente une sortie dont l'ouverture est contrôlable par une électrovanne pour réduire la pression dans l'enceinte.

On peut aussi prévoir que chaque enceinte 600, 600' qui loge une poche de décharge 60, 60' soit raccordée à un générateur de vide 80. Le générateur de vide 80 et/ou les moyens de mise sous pression 70 permettent d'appliquer une pression ou une dépression à la poche de décharge 60 ou 60', de manière à ajuster la pression moyenne dans le compartiment de dialysat 14, par exemple lors d'une phase d'ultrafiltration. Dans ce cas, l'organe aval C6 ou C6' de la poche de décharge 60 ou 60' qui est utilisée pour ajuster la pression moyenne dans le compartiment de dialysat 14, est fermé tandis que l'organe amont C5 ou C5' correspondant est ouvert.

Ledit générateur de vide 80 peut être commun pour la mise en dépression des enceintes du système d'amenée 5 de dialysat et la mise en dépression des enceintes du système d'évacuation 6.

Ladite machine comprend des moyens de détermination de la quantité de dialysat et de liquide récupérée dans la poche de décharge 60 ou 60' qui se vide.

Selon un mode de réalisation, lesdits moyens de détermination de la quantité de dialysat et de liquide récupérée dans la ou les poches de décharge 60, 60' comprennent une restriction de passage 620, ménagée dans la ligne 62 d'évacuation de dialysat et située en aval desdites poches de décharge 60, 60', et des moyens de mesure de la différence de pression aux bornes de ladite restriction de passage 620.

Lesdits moyens de mesure de la différence de pression aux bornes de la restriction 620 de la ligne d'évacuation 62 peuvent être similaires à ceux associés à la restriction 520 de la ligne d'amenée 52.

Ainsi, lesdits moyens de mesure de la différence de pression aux bornes de la restriction 620 de la ligne d'évacuation 62 peuvent comprendre deux orifices de prise de pression ménagés, dans la paroi périphérique de ladite ligne 62, l'un en amont et l'autre en aval de ladite restriction 620. Selon un aspect particulier, chaque orifice de prise de pression est associé à un capteur de pression agencé de manière à mesurer la pression dans la ligne 62 au niveau dudit orifice tout en étant écarté dudit orifice pour ne pas être en contact avec le dialysat circulant dans la ligne 62.

De manière similaire aux moyens de mesure de la différence de pression aux bornes de la restriction 520, un filtre perméable à l'air et imperméable aux agents infectieux et aux liquides, peut être interposé entre chaque capteur de pression 623, 624 et l'orifice de prise de pression correspondant. En particulier, chaque capteur de pression 623, 624 est monté dans une cavité du bâti de la machine à l'aide d'une pièce support creuse qui est destinée à être couplée au conduit 621, 622 relié à l'orifice de prise de pression correspondant.

Les capteurs de pression 623, 624 associés à ladite restriction 620 permettent de déterminer la différence de pression aux bornes de la restriction 620 et ainsi le débit de dialysat et de liquide évacué de la poche de décharge 60 ou 60', ce qui permet de déterminer, en mesurant le temps d'écoulement correspondant, la quantité de dialysat et de liquide récupérée dans ladite poche de décharge 60 ou 60'.

### Source d'alimentation

La source d'alimentation en dialysat utilisée pour une séance donnée de dialyse est de préférence une ou un ensemble de poches nourricières de plus grand volume, par exemple 5L, que celui, par exemple 150 ml, de ladite poche ventricule 50, 50'.

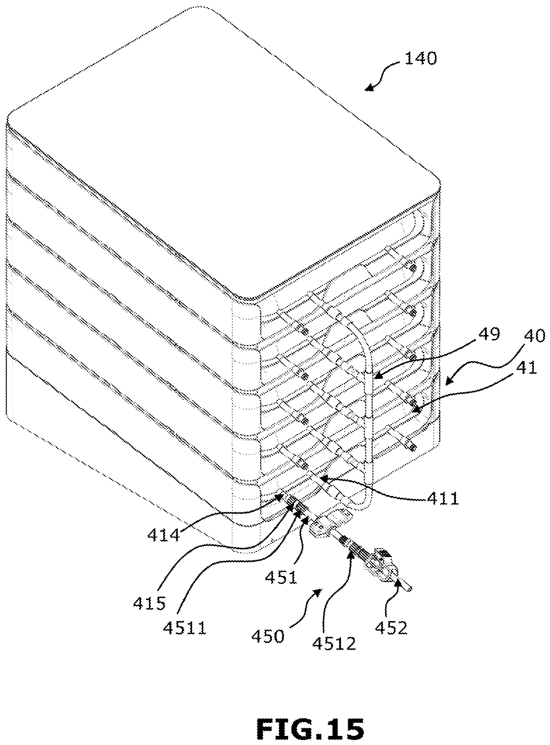

Selon un mode de réalisation et comme illustré à la Figure 15, chaque source d'alimentation 40, 40' comprend une pluralité de poches 41, dites poches nourricières, et un dispositif de raccordement 49, appelé pieuvre d'interconnexion, qui comprend une pluralité de conduites permettant de raccorder les poches nourricières entre elles au niveau d'embouts 411, de préférence à l'aide de raccords rapides, par exemple des raccords de type Luer Lock. Selon un mode de réalisation, chaque source 40 ou 40' d'alimentation en dialysat peut être logée dans une structure support 140 à étages. Un exemple de structure support à étages est décrit dans la demande internationale WO2014001680. Ainsi comme illustré à la Figure 15, chaque poche nourricière 41 de la source d'alimentation est positionnée dans un étage de la structure support 140.

La source 40 d'alimentation comprend une conduite 414 munie d'un connecteur (ou raccord) 415 qui peut être connecté au et déconnecté du premier tronçon 451. La source 40' d'alimentation comprend une conduite 414' munie d'un connecteur 415' (de préférence identique ou similaire au connecteur 415) qui, à l'état retiré du premier tronçon 451, peut être connecté au et déconnecté du deuxième tronçon 452 du système de raccordement 450 présenté ci-dessous. Préférentiellement, la conduite 414 est située au niveau de la poche nourricière 41 la plus basse à l'état positionné des poches nourricières dans la structure support 140. En vue d'une première séance, le connecteur 415 de la source d'alimentation 40 est connecté au premier tronçon 451. Préférentiellement, le connecteur 415 est indémontable, par exemple soudé, à la conduite 414 de la source d'alimentation 40. Le retrait du premier tronçon 451 par rapport au deuxième tronçon 452, permet de connecter le connecteur 415' (du même type que le connecteur 415) de la source d'alimentation 40' au deuxième tronçon 452. Ledit connecteur 415' est aussi déconnectable du deuxième tronçon 452 et est de préférence solidaire de manière indémontable de la conduite 414' de la source d'alimentation 40'.

### Système de raccordement de la source d'alimentation

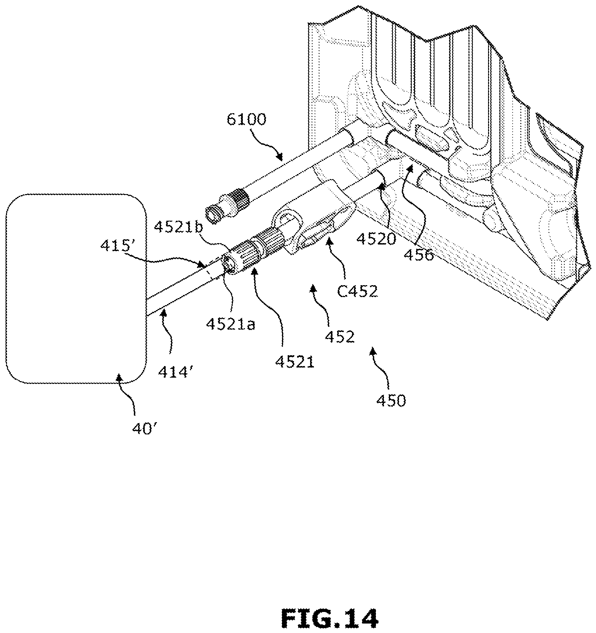

La machine comprend un système de raccordement 450 entre une source 40 d'alimentation en dialysat et la ligne d'amenée 52 de dialysat, en particulier au niveau d'un raccord 456 (Figure 14) qui communique avec l'entrée 511 du système de délivrance 51 de dialysat, pour approvisionner en dialysat les poches du système 51 de délivrance de dialysat. Dans l'exemple illustré aux Figures 2 et 4, la source d'alimentation en dialysat utilisée pour une première séance de dialyse est référencée 40 et celle, distincte de la source d'alimentation 40, utilisée pour une deuxième séance de dialyse est référencée 40'.

La source d'alimentation en dialysat utilisée pour une séance donnée de dialyse est de préférence une ou un ensemble de poches nourricières de plus grand volume, par exemple 5L, que celui, par exemple 150 ml, de ladite poche ventricule 50, 50'.

Comme illustré aux Figures 2 à 4 7, 9, 11 et 14, le système de raccordement 450 comprend un premier tronçon 451 qui comprend un conduit 4510 présentant une extrémité apte à être raccordée par un connecteur 4511, de préférence un connecteur de type Luer Lock, par exemple mâle, à un connecteur 415 correspondant, par exemple femelle, dont est munie une conduite 414 de la première source d'alimentation en dialysat 40. Le raccord de type Luer Lock correspond à la norme NF EN ISO 594.

Ledit connecteur 4511 comprend :
- un élément tubulaire 4511a apte à être engagé dans une ouverture du raccord (connecteur) correspondant de la source d'alimentation en dialysat pour permettre une communication de liquide à l'intérieur dudit élément tubulaire ; et
- une paroi périphérique 4511b qui s'étend autour et à écartement de l'élément tubulaire 4511a pour définir un espace annulaire d'insertion d'une paroi périphérique dudit raccord de la source d'alimentation en dialysat.

Le conduit 4510 du premier tronçon 451 comprend à l'autre extrémité un connecteur 4512 configuré pour être connecté à un connecteur d'un deuxième tronçon 452 du système de raccordement 450.

Selon un mode de réalisation et comme illustré à la Figure 9, le premier tronçon 451 est muni d'un système C451 de pince, initialement à l'état ouvert et qui est actionnable pour se refermer sur le conduit 4510, en dégradant le conduit 4510 et/ou en étant verrouillé en position fermée. Le système C451 est aussi appelé organe de fermeture inviolable. L'utilisation d'un tel organe C451 de fermeture inviolable permet de s'assurer que le premier tronçon 451, en particulier le conduit 4510 correspondant, ne soit pas réutilisé.

Le système de raccordement 450 comprend aussi un deuxième tronçon 452 qui comprend un conduit 4520 présentant une extrémité apte à être raccordée à une deuxième source d'alimentation en dialysat 40'.

A cet effet ladite extrémité du deuxième tronçon 452 est munie d'un connecteur 4521, par exemple de type Luer Lock mâle.

Le connecteur 4521 comprend :
- un élément tubulaire 4521a apte à être engagé dans une ouverture deuxième connecteur 4512 du premier tronçon 451 pour permettre une communication de liquide à l'intérieur dudit élément tubulaire ; et
- une paroi périphérique 4521b qui s'étend autour et à écartement de l'élément tubulaire 4521a pour définir un espace annulaire d'insertion de la paroi périphérique dudit connecteur de la source d'alimentation en dialysat.

Préférentiellement, l'autre extrémité du deuxième tronçon 452 est connectée à un raccord 456 qui est en communication fluidique avec l'entrée 511 du système de délivrance 51.

Selon un mode de réalisation préférentiel, le deuxième tronçon 452 est équipé d'un système C452 de pince pour l'ouverture/fermeture du deuxième tronçon 452.

Pour une première séance de dialyse et Comme illustré à la Figure 9, la source 40 d'alimentation en dialysat est connectée au premier tronçon 451 du système de raccordement 450, par sa conduite 414 munie d'un connecteur adapté à être connecté au connecteur 4511 du premier tronçon 451. Le connecteur de la conduite 414 est de préférence un connecteur Luer Lock femelle, par exemple un connecteur du type du connecteur référencé 4512, 821 ou 921.

Durant cette première séance de dialyse, le système de pince C451 est laissé à l'état ouvert, de même que le système de pince C452 pour permettre au dialysat provenant de ladite source d'alimentation d'alimenter le système de délivrance 51 qui est raccordé à la ligne d'amenée 52. Le dialysat peut ainsi s'écouler de la source 40 d'alimentation vers le système de délivrance 51 en passant à travers le premier tronçon 451 et le deuxième tronçon 452.

Avantageusement, les conduites 4510, 4520 du système de raccordement 450 se présentent sous forme de tuyaux souples pour pouvoir être pincées. Les conduites 4510, 4520 présentent à l'état non pincé (ou à l'état non écrasé) une section de diamètre (extérieur et intérieur) constant sur toute leur longueur. Autrement dit, les conduites 4510, 4520 ne présentent pas de réserves ou poches (par exemple de liquide) intermédiaires entre leurs extrémités.

L'entrée 511 d'approvisionnement du système 51 de délivrance de dialysat peut correspondre à un nœud de liaison entre les entrées des poches 50, 50'.

Après la première séance de dialyse donnée, le système C451 est fermé par l'opérateur (l'utilisateur ou un tiers) pour rendre le premier tronçon 451 inutilisable, et le premier tronçon 451 est déconnecté du deuxième tronçon 452 (déconnexion du connecteur 4512 par rapport au connecteur 4521) pour être jeté.

Puis pour une deuxième séance de dialyse donnée, le deuxième tronçon 452 est raccordé à une autre source d'alimentation en dialysat 40' dont les caractéristiques peuvent être similaires ou identiques à celles de la source d'alimentation en dialysat 40.

Autrement dit, la source d'alimentation 40 est retirée avec le tronçon 452 auquel la source d'alimentation 40 était connectée et la source d'alimentation 40' est connectée au tronçon 451 suivant du système de raccordement 450.

On comprend que le système de raccordement 450 peut présenter un plus grand nombre de tronçons successifs de manière à permettre de réaliser autant de séances de dialyse que de tronçons en retirant après chaque séance de dialyse le tronçon utilisé pour pouvoir utiliser le tronçon suivant pour la prochaine séance de dialyse. A cet effet, on peut prévoir que le deuxième tronçon soit luimême formé d'une pluralité de tronçons (sous-tronçons) connectés en série.

On comprend que l'approvisionnement des poches 50, 50' se fait au moyen de la source 40 ou 40' d'alimentation en dialysat qui est à l'extérieur des enceintes, en particulier à l'extérieur de la cassette 9.

Comme rappelé ci-avant et illustré à la Figure 15, chaque source d'alimentation 40 ou 40' comprend une pluralité de poches 41, dites poches nourricières.

Comme rappelé ci-dessus, chaque tronçon 451 ou 452 du système de raccordement 450 peut ainsi être, sélectivement, connecté sur une conduite 414 de la poche nourricière 41 la plus basse à l'état positionné des poches nourricières dans la structure support 140.

Une telle utilisation combinée du système de raccordement 450 et du système de délivrance 51 de dialysat à poche souple, évite d'avoir à ouvrir les enceintes 500, 500', et donc d'accéder à la cassette, non seulement au cours d'une séance de dialyse, mais également entre deux séances successives tant qu'il reste un tronçon non encore utilisée du système de raccordement. En effet, au cours d'une séance avec la source d'alimentation 40 puis au cours de la séance suivante avec la source d'alimentation 40', la cassette et donc les poches ventricules peuvent être laissées dans la machine sans avoir à être remplacées.

### Cassette

Comme illustré à la Figure 7, une partie de la machine se présente sous la forme d'une cassette 9 présentant un corps 900. La cassette 9 est une cassette amovible par rapport au bâti (ou châssis) de la machine qui est destinée à être insérée dans un logement dudit bâti. Ladite cassette 9 comprend notamment la ou lesdites poches 50, 50', un corps 900, et la ligne d'amenée 52. Selon un aspect particulier, la cassette 9 comprend aussi la ligne d'évacuation 62 de dialysat, et la ou les poches de décharge 60, 60' du système d'évacuation.

Dans l'exemple illustré aux figures, ladite cassette 9 comprend notamment les lignes de dialysat 52, 62, et chaque poche 50, 50', 60, 60'. La cassette peut comprendre le système de raccordement 450 de la source 40, 40' d'alimentation en dialysat au système de délivrance 51 et/ou le système de raccordement 800, 900 au dialyseur.

Selon un mode de réalisation, les capteurs de pression 523, 524, 623, 624, représentés aux Figures 2 à 4, sont fixés au bâti de la machine. A l'état inséré de la cassette 9 dans la machine, les conduits de prise de pression sont en communication pneumatique avec les capteurs de pression. Par communication pneumatique on entend que le capteur et le conduit de prise de pression correspondant sont agencés l'un par rapport à l'autre de telle sorte que le capteur est apte à mesurer la pression qui règne dans ledit conduit, et donc la pression qui règne dans la ligne au niveau où est ménagé l'orifice de prise de pression correspondant.

### Unité de pilotage

Comme évoqué précédemment, ladite machine comprend aussi une unité de pilotage 10, telle qu'un automate programmable. L'unité de pilotage peut être configurée pour exécuter des étapes de dialyse, telles que décrites dans les demandes internationales WO2013050689A1 ou WO2019150058A2.

Une séance de dialyse comprend, outre des phases d'amorçage et de restitution, une phase de dialyse qui peut comprendre une pluralité d'étapes d'ultrafiltration en alternance avec des étapes de rétrofiltration. Un exemple de fonctionnement de la machine lors d'une séance de dialyse avec une pluralité d'étapes d'ultrafiltration en alternance avec des étapes de rétrofiltration est décrit dans la demande internationale WO2013050689A1 ou WO2019150058A2. Cependant, il convient de noter que la phase de dialyse comprend de manière générale une phase d'échange entre le compartiment sang et le compartiment dialysat, par exemple par osmose, et peut ne pas comprendre de phase de rétrofiltration et/ou de phase d'ultrafiltration.

Pour la phase de dialyse, l'unité de pilotage 10 commande en particulier les moyens de mise sous pression des poches 50, 50' et les systèmes à pince C1,C2; C1',C2' et C5,C6 ; C5',C6' de fermeture/ouverture du système d'amenée 5 et du système d'évacuation 6. Dans une séance de dialyse, la phase d'amorçage correspond à l'arrivée du sang dans le compartiment sang du dialyseur. Pour cette phase d'amorçage, l'unité de pilotage commande la pompe à sang Psg.

La séance de dialyse se termine par une phase de restitution au cours de laquelle le sang présent dans le circuit sang (ligne veineuse, ligne artérielle, et compartiment sang du patient) est restitué au patient. Pour cette phase de restitution, l'unité de pilotage commande également la pompe à sang Psg, une poche de sérum physiologique étant connectée à la ligne L1 pour permettre de restituer le sang sans risque d'introduction d'air.

Selon un mode de réalisation, l'unité de pilotage 10 est en outre configurée pour mettre en œuvre un procédé de rinçage et d'élimination d'air du circuit extracorporel de la machine de dialyse, par exemple en exécutant des étapes de rinçage et/ou d'élimination d'air, telles que décrites dans la demande FR 2001604, déposée le 18 février 2020 et non encore publiée.

Ladite unité de pilotage 10 se présente sous la forme d'un système électronique et informatique qui comprend par exemple un processeur, tel qu'un microprocesseur, une mémoire de travail et une mémoire de données. Ladite unité de pilotage peut se présenter sous la forme d'un microcontrôleur.

Autrement dit, les fonctions et étapes décrites peuvent être mise en œuvre sous forme de programme informatique ou via des composants matériels (p. ex. des réseaux de portes programmables). En particulier, les fonctions et étapes opérées par l'unité de pilotage ou ses modules peuvent être réalisées par des jeux d'instructions ou modules informatiques enregistrés dans une mémoire pour leur exécution par un processeur ou contrôleur ou être réalisées par des composants électroniques dédiés ou des composants de type FPGA ou ASIC. Il est aussi possible de combiner des parties informatiques et des parties électroniques.

Lorsqu'il est précisé que l'unité ou des moyens ou modules de ladite unité sont configurés pour réaliser une opération donnée, cela signifie que l'unité comprend des instructions informatiques et les moyens d'exécution correspondants qui permettent de réaliser ladite opération et/ou que l'unité comprend des composants électroniques correspondants.

Ladite unité de pilotage présente aussi une interface d'entrée de données. On peut prévoir que ladite interface permette d'entrer des données relatives au patient et/ou aux consommables de la machine.

### Autres composants ou moyens

Dans l'exemple illustré aux figures, la machine de dialyse est aussi munie de composants classiques pour assurer un traitement fiable et efficace du liquide corporel à traiter, notamment un détecteur de fuite de sang FS ménagé dans la ligne d'évacuation 62. Avantageusement, l'unité de pilotage 10 est configurée pour permettre d'étalonner les débitmètres l'un par rapport à l'autre.

Chaque enceinte 500, 500' est munie de moyens de chauffage ou préchauffage de l'enceinte. Chaque enceinte 500, 500', 600, 600' est aussi munie d'une vanne de mise à l'air V2, V5, V8, V11 associée à un filtre à air F1, F2, F3, F4, et d'un capteur de pression P7, P8, P9 et P10.

Les moyens de mise sous pression 70 comprennent les éléments suivants :
un réservoir d'air comprimé R1, un capteur de pression P11 du réservoir R1, un compresseur Pa1, un clapet anti-retour pour l'air comprimé Ar1, et un filtre à air et silencieux pour l'air comprimé Si1.

Le générateur de vide 80 comprend un réservoir de vide d'air R2, un capteur de pression P12 du réservoir R2, une pompe à vide Pa2, un clapet anti-retour pour le vide d'air Ar2 et un filtre à air et silencieux pour le vide d'air Si2.

La ligne d'amenée 52 est aussi munie de moyens de chauffage Ch3 du dialysat associé à des moyens de mesure de température T1, T2, T3. La ligne d'évacuation 62 est munie de moyens de mesure de température T4, T5 en aval des poches 60, 60'.

Avantageusement, la ligne veineuse L2 comprend aussi un organe de fermeture/ouverture CV, tel qu'une pince. La machine peut comprendre une ligne de by-pass L10, ainsi qu'un ou plusieurs organes Vbp de fermeture/ouverture associés. La ligne de by-pass L10 peut être utilisée pour réaliser l'amorçage et la calibration du circuit de dialysat, et pour permettre de ne pas injecter dans le dialyseur du dialysat défectueux, par exemple du fait d'un problème de température.

Avantageusement, la ligne d'évacuation du dialyseur est raccordée à un récipient de récupération ou à un système de mise à l'égout Egt.

### Procédé

Selon un mode de réalisation, la mise en œuvre d'une séance de dialyse avec une machine de dialyse telle que décrite ci-dessus comprend les étapes décrites ci-après. Comme rappelé ci-dessus, lorsque cela est techniquement admissible, l'ordre de certaines étapes peut être inversé et le procédé peut comprendre entre deux étapes décrites d'autres étapes supplémentaires. Le procédé décrit ci-dessous permet l'utilisation prolongée d'une cassette après l'achèvement d'une première séance de dialyse pour l'exécution d'une deuxième séance de dialyse avec la même cassette, avec un risque réduit de contamination lors de l'intervention de l'opérateur pour changer le dialyseur et/ou changer la source d'alimentation en dialysat.

En vue d'une première utilisation d'une cassette 9, à l'étape 1600, l'utilisateur ouvre une porte de la machine de dialyse pour introduire dans un logement correspondant la cassette 9 (par exemple visible à la Figure 7).

La présence des bouchons 829 et 929 permet de limiter le risque de contamination des connecteurs 820 et 920 lorsque la cassette est sortie de son emballage pour la première séance et avant montage dans la machine et avant connexion au dialyseur.

Comme illustré aux Figures 2 et 8, l'utilisateur ouvre les bouchons 829 et 929 et connecte la ligne d'amenée 52 à l'entrée 401 de la zone de passage de dialysat 14 du dialyseur 100 avec le système de raccordement 800 et connecte la ligne d'évacuation 62 à la sortie 402 de la zone de passage de dialysat 14 du dialyseur 100 avec le système de raccordement 900 (étape 1610).

Les systèmes de pince C451, C452 sont en position d'ouverture, de sorte que les conduites 451, 452 ne sont pas pincées.

L'utilisateur met en place des poches nourricières 41 de dialysat de la source d'alimentation 40 dans la structure support 140. L'utilisateur raccorde les poches nourricières 41 entre-elles à l'aide de la pieuvre d'interconnexion 49.

L'utilisateur installe et connecte une poche de sérum physiologique sur la ligne L1 en amont de la pompe Psg.

L'utilisateur connecte les lignes veineuse L2 et artérielle L1 du circuit extracorporel au dialyseur 100.

En vue d'une première séance de dialyse et comme illustré aux Figures 2 et 9, l'utilisateur connecte si besoin le premier tronçon 451 du système de raccordement 450 à la source d'alimentation 40 (étape 1620). Selon un mode de réalisation préféré, le système de raccordement 450 est fourni (livré) dans une configuration où le premier tronçon 451 et le deuxième tronçon 452 sont déjà raccordés l'un à l'autre, le deuxième tronçon 452 étant aussi raccordé au système de délivrance 51. Le système de pince C452 du deuxième tronçon est laissé ouvert pour permettre au dialysat de la source d'alimentation 40 d'alimenter le système de délivrance 51 par l'intermédiaire des tronçons 451 et 452 du système de raccordement. A noter que le système de pince C451 est aussi laissé ouvert tant que la première séance de dialyse n'est pas terminée.

Ainsi, pour cette première séance de dialyse, le connecteur 4511 du premier tronçon 451 est raccordé au connecteur 415 de la première source d'alimentation 40 en dialysat, tandis que le deuxième tronçon 452 est en communication fluidique avec le premier tronçon 451 via les connecteurs 4512, 4521. Les systèmes C451, C452 de pince sont en position d'ouverture.

L'utilisateur met la machine sous tension pour le démarrage de la machine. L'unité de pilotage acquière par exemple par saisie dans une interface de données et/ou par lecture dans une mémoire de données relatives aux consommables ou à leur lot, telles que des données d'identification de la source d'alimentation en dialysat, du dialyseur, du circuit extracorporel, et de la cassette.

L'utilisateur peut effectuer des paramétrages à l'aide de l'interface de saisie, notamment en saisissant les données de poids "sec" et réel pour permettre à l'unité de pilotage de déterminer la quantité de fraction aqueuse en excès à retirer du liquide corporel.

Lorsque les conditions d'exécution d'une séance de dialyse sont remplies, comme expliqué ci-dessus, l'unité de pilotage peut commander l'exécution d'une première séance de dialyse comprenant des séquences d'amorçage, de dialyse et de restitution. La séquence d'amorçage peut être précédée d'une étape de rinçage des consommables en faisant circuler du dialysat dans le circuit dialysat, en particulier dans la cassette. En parallèle on fait circuler du sérum physiologique dans le circuit extracorporel (lignes de sang et compartiment sang) pour rincer ce circuit extracorporel. Le patient peut alors se connecter à la ligne L1.

A la fin de la première séance, l'unité de pilotage commande la purge du dialysat présent dans le système d'amenée 5 et le système d'évacuation 6.

La machine peut alors être mise en veille potentiellement de manière intermittente.

Après la première séance de dialyse, l'utilisateur retire certains éléments consommables de la machine, tels que des lignes artérielle L1 et veineuse L2 ; et le dialyseur. La cassette 9 reste dans la machine à l'exception des raccords 820 et 920 que l'opérateur déconnecte de la ligne d'amenée 52 et de la ligne d'évacuation 62 par rapport au dialyseur 100, comme illustré à la Figure 10, pour les remplacer par de nouveaux raccords 820' et 920' qui sont connecté respectivement aux raccords 810 et 910 comme illustré aux Figures 3, 4 et 12, les bouchons 829' et 929' étant initialement en position fermée.

L'utilisateur retire aussi la première source d'alimentation 40 (étape 1630). Préférentiellement, le système à pince C451 inviolable est fermé après la première séance, avant ou après retrait de la source d'alimentation.

Préférentiellement, l'extrémité du système de raccordement formée par le connecteur 4511 est fermée par un bouchon qui peut être un bouchon 4519 présent initialement sur ce connecteur 4511, et qui a précédemment été retiré pour pouvoir connecter la première source d'alimentation 40, avant de procéder à la première séance de dialyse.

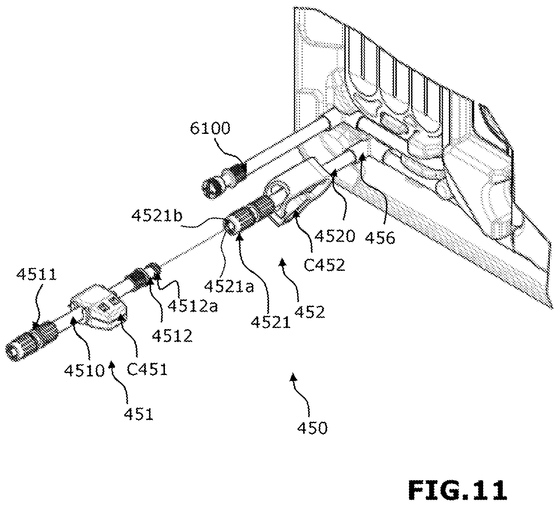

Pour la mise en œuvre d'une deuxième séance de dialyse, à l'étape 1640, le premier tronçon 451 du système de raccordement 450 est retiré, comme illustré à la Figure 11, ce qui laisse libre le connecteur 4521 du deuxième tronçon 452 pour pouvoir y raccorder le connecteur 415' d'une autre source d'alimentation 40'.

Le retrait du premier tronçon 451 est réalisé de préférence juste avant la connexion du deuxième tronçon 452 du système de raccordement 450 à la deuxième source d'alimentation en dialysat 40'.

Ainsi, pour la mise en œuvre d'une deuxième séance de dialyse et comme illustré à la Figure 14, le deuxième tronçon 452 du système de raccordement 450 est raccordé à la deuxième source d'alimentation en dialysat 40' (étape 1650). Selon un mode de réalisation, comme pour la source d'alimentation 40, les poches nourricières de la source d'alimentation 40' sont raccordées entre-elles à l'aide d'une pieuvre d'interconnexion. L'utilisateur ouvre le système C452 de pince (qui peut avoir été précédemment fermé après la première séance de dialyse) pour libérer la communication fluidique à l'intérieur de la deuxième branche 452.

De nouvelles lignes veineuse L2' et artérielle L1' sont connectées à un nouveau dialyseur 100'. L'utilisateur installe et connecte une nouvelle poche de sérum physiologique.

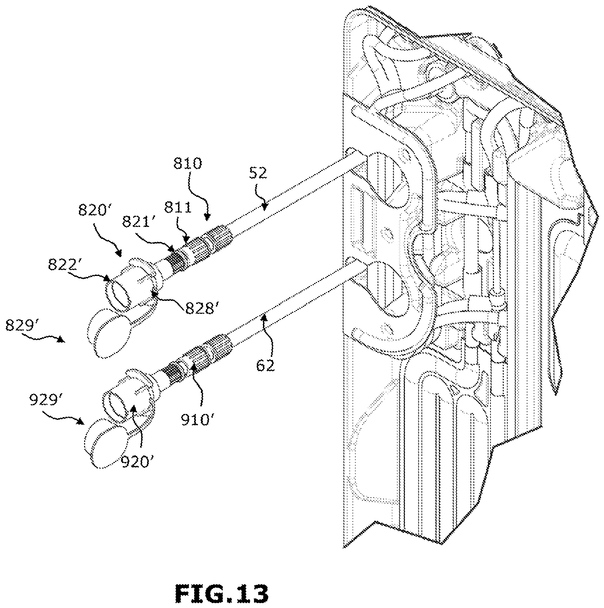

Les bouchons 829', 929' des nouveaux connecteurs 820', 920' sont alors ouverts, comme illustré à la Figure 13, pour connecter la ligne d'amenée 52 à l'entrée 401 de la zone de passage de dialysat 14 d'un nouveau dialyseur 100' et la ligne d'évacuation 62 à la sortie 402 de la zone de passage de dialysat 14 du nouveau dialyseur 100'.

Comme pour la première séance, l'utilisateur peut saisir des paramètres à l'aide de l'interface de saisie en vue de la deuxième séance de dialyse.

Lorsque les conditions d'exécution d'une nouvelle séance de dialyse sont remplies, comme expliqué ci-dessus, l'unité de pilotage 10 peut commander l'exécution d'une deuxième séance de dialyse comprenant des séquences d'amorçage, de dialyse et de restitution.

Les bouchons 829' et 929' présents sur les parties 820' et 920', disponibles pour la deuxième séance, permettent de protéger ces dernières des contaminations lorsqu'elles sont sorties de leur emballage unitaire avant le démarrage de la deuxième séance.

L'invention n'est pas limitée aux modes de réalisation illustrés dans les dessins, mais définie par les revendications annexées.

De plus, le terme « comprenant » n'exclut pas d'autres éléments ou étapes. En outre, des caractéristiques ou étapes qui ont été décrites en référence à l'un des modes de réalisation exposés ci-dessus peuvent également être utilisées en combinaison avec d'autres caractéristiques ou étapes d'autres modes de réalisation exposés ci-dessus.

## Revendications

1. Machine de dialyse permettant de traiter un liquide corporel, tel que du sang ou du plasma, la machine de dialyse comportant :
- un dialyseur (100, 100') comprenant une enceinte qui inclut une zone de passage du liquide corporel (12) qui présente une entrée (201) de liquide corporel et une sortie (202) de liquide corporel, et une zone de passage de dialysat (14) qui présente une entrée de dialysat (401) et une sortie de dialysat (402), et un système de membrane (3) entre la zone de passage de liquide corporel et la zone de passage de dialysat (14) ;
- un système de délivrance (51) de dialysat,
- un système de raccordement (450) permettant de raccorder une source d'alimentation en dialysat (40) au système de délivrance (51) de dialysat, le système de raccordement (450) comprenant :
- un premier tronçon (451), qui comporte un conduit (4510) muni à une extrémité d'un premier dispositif de raccordement (4511) qui est connectable à un connecteur (415) d'une première source d'alimentation en dialysat (40), et qui est déconnectable dudit connecteur (415) de la première source d'alimentation en dialysat (40) et muni à l'autre extrémité d'un deuxième dispositif de raccordement (4512), ledit conduit (4510) étant un conduit souple de section constante à l'état non écrasé dudit conduit (4510), et
- un deuxième tronçon (452), qui comporte un conduit (4520) qui présente une extrémité raccordée au système de délivrance (51) de dialysat et qui est muni à une extrémité opposée d'un dispositif de raccordement (4521) connecté, de manière déconnectable, avec le deuxième dispositif de raccordement (4512) du premier tronçon (451), pour fournir une connexion en série du premier tronçon (451) et du deuxième tronçon (452), tout en permettant de déconnecter ledit premier tronçon (451) du deuxième tronçon (452), ledit conduit (4520) du deuxième tronçon (452) étant un conduit souple de section constante à l'état non écrasé dudit conduit (4520),
de sorte que, à l'état déconnecté et retiré dudit premier tronçon (451) par rapport au deuxième tronçon (452), ledit dispositif de raccordement (4521) du deuxième tronçon (452) est raccordable à un connecteur (415') d'une deuxième source d'alimentation en dialysat (40') et déconnectable dudit connecteur (415') de la deuxième source d'alimentation en dialysat (40').

2. Machine selon la revendication 1, dans laquelle le système de raccordement comprend un élément (C451) de fermeture permettant, à l'état fermé, d'empêcher la circulation de liquide à l'intérieur du conduit (4510) du premier tronçon (451).

3. Machine selon la revendication 2, dans laquelle l'élément (C451) de fermeture est de type inviolable de sorte que, à l'état fermé, l'élément (C451) de fermeture est verrouillé et/ou dégrade le conduit (4510) du premier tronçon (451) pour empêcher la réutilisation du conduit après que l'élément (C451) de fermeture a été fermé.

4. Machine selon l'une quelconque des revendications précédentes, dans laquelle le deuxième tronçon (452) comprend un élément (C452) d'ouverture et de fermeture activable et désactivable permettant d'autoriser ou d'empêcher la circulation de liquide à travers le conduit du deuxième tronçon (452).

5. Machine selon l'une quelconque des revendications précédentes, dans laquelle le premier dispositif de raccordement (4511) du premier tronçon (451) comprend :
- un élément tubulaire (4511a) apte à être engagé dans une ouverture du connecteur (415) correspondant de la première source d'alimentation (40) en dialysat pour permettre une communication de liquide à l'intérieur dudit élément tubulaire (4511a) ; et
- une paroi périphérique (4511b) qui s'étend autour et à écartement de l'élément tubulaire (4511a) pour définir un espace annulaire d'insertion d'une paroi périphérique dudit connecteur de la première source d'alimentation en dialysat.

6. Machine selon l'une quelconque des revendications précédentes, dans laquelle le dispositif de raccordement (4521) du deuxième tronçon (452) comprend :
- un élément tubulaire (4521a) qui est apte à être engagé dans une ouverture du deuxième dispositif de raccordement (4512) du premier tronçon (451) pour permettre une communication de liquide à l'intérieur dudit élément tubulaire (4521a), et qui est, à I 'état retiré du premier tronçon (451), apte à être engagé dans une ouverture du connecteur (415') de la deuxième source d'alimentation (40') en dialysat pour permettre une communication de liquide à l'intérieur dudit élément tubulaire (4521a); et
- une paroi périphérique (4521b) qui s'étend autour et à écartement de l'élément tubulaire (4521a) pour définir un espace annulaire d'insertion d'une paroi périphérique dudit deuxième dispositif de raccordement (4512) du premier tronçon (4521) ou, à I 'état retiré du premier tronçon (451), pour définir un espace annulaire d'insertion d'une paroi périphérique du connecteur (415') de la deuxième source d'alimentation en dialysat (40').

7. Machine selon l'une quelconque des revendications précédentes, dans laquelle le deuxième dispositif de raccordement (4512) du premier tronçon (451) comprend une paroi périphérique munie d'un filetage (4512a) apte à coopérer avec un taraudage ménagé dans la paroi périphérique (4521b) du dispositif de raccordement (4521) du deuxième tronçon (452).

8. Machine selon l'une quelconque des revendications précédentes, dans laquelle la machine comprend :
- une ligne d'amenée (52) de dialysat qui est raccordable à une entrée (401) d'une zone de passage de dialysat (14) du dialyseur (100), et
- une ligne d'évacuation (62) de dialysat qui est raccordable à une sortie (402) de la zone de passage de dialysat (14) du dialyseur (100).

9. Machine selon la revendication 8, dans laquelle la ligne d'évacuation (62) comprend une conduite (6100) pour le raccordement à un dispositif d'évacuation tel qu'un système de mise à l'égout (Egt) et/ou un système de récupération du dialysat usagé.

10. Machine selon l'une quelconque des revendications précédentes, dans laquelle la machine comprend au moins une poche (50) souple, appelée poche ventricule, destinée à contenir du dialysat, formant partie dudit système de délivrance (51).

11. Machine selon la revendication 10, dans laquelle ladite au moins une poche est inclue dans un dispositif, appelé cassette (9), qui est insérable et retirable d'un logement correspondant ménagé dans la machine de dialyse.

12. Machine de dialyse selon la revendication 11, dans laquelle la machine comprend un châssis et ledit dispositif, appelé cassette, est amovible par rapport au châssis de la machine.

13. Procédé pour raccorder successivement des sources d'alimentation (40, 40') en dialysat au système de raccordement (450) d'une machine selon l'une quelconque des revendications 1 à 12, le procédé comprenant les étapes suivantes :
- fournir le système de raccordement (450) dans une configuration où le premier tronçon (451) est raccordé au deuxième tronçon (452), le deuxième tronçon étant raccordé par son autre extrémité au système de délivrance (51) de dialysat (5) ;
- fournir une première source d'alimentation en dialysat (40) présentant un connecteur (415) non raccordé au système de raccordement (450) ;
- lorsqu'un bouchon (4519) ferme l'extrémité du premier tronçon (451) opposée à celle raccordée au deuxième tronçon (452), retirer ledit bouchon (4519);
- raccorder le premier dispositif de raccordement (4511) du premier tronçon (451) à la première source d'alimentation en dialysat (40), et après usage de la première source d'alimentation (40) en dialysat :
- de préférence, obturer le premier tronçon (451), par exemple par fermeture d'un système de pince (C451) sur le premier tronçon,
- de préférence, retirer la première source d'alimentation (40) en dialysat et fermer le dispositif de raccordement (4511) du premier tronçon (451) à l'aide d'un bouchon (4519) ;
- retirer le premier tronçon (451) par rapport au deuxième tronçon (452) du dispositif de sorte que le deuxième tronçon (452) présente un dispositif de raccordement (4521) libre;
- fournir une deuxième source d'alimentation en dialysat (40') présentant un connecteur (415') non raccordé au système de raccordement (450) ;
- raccorder le connecteur (415') de la deuxième source d'alimentation en dialysat (40') au dispositif de raccordement (4521) libre dudit deuxième tronçon (452).

## Patentansprüche

1. Dialysemaschine, die gestattet, eine Körperflüssigkeit wie Blut oder Plasma zu behandeln, wobei die Dialysemaschine aufweist:
- einen Dialysator (100, 100'), umfassend ein Gehäuse, das einen Körperflüssigkeitsdurchgangsbereich (12) einschließt, der einen Körperflüssigkeitseinlass (201) und einen Körperflüssigkeitsauslass (202) aufweist, und einen Dialysatdurchgangsbereich (14), der einen Dialysateinlass (401) und einen Dialysatauslass (402) aufweist, und ein Membransystem (3) zwischen dem Körperflüssigkeitsdurchgangsbereich und dem Dialysatdurchgangsbereich (14);
- ein Dialysatabgabesystem (51),
- ein Verbindungssystem (450), das gestattet, eine Dialysatversorgungsquelle (40) mit dem Dialysatabgabesystem (51) zu verbinden, wobei das Verbindungssystem (450) umfasst:
- einen ersten Abschnitt (451), der eine Leitung (4510) aufweist, die an einem Ende mit einer ersten Verbindungsvorrichtung (4511) versehen ist, die mit einem Verbinder (415) einer ersten Dialysatversorgungsquelle (40) verbindbar ist, und die von dem Verbinder (415) der ersten Dialysatversorgungsquelle (40) trennbar ist und am anderen Ende mit einer zweiten Verbindungsvorrichtung (4512) versehen ist, wobei die Leitung (4510) eine flexible Leitung mit konstantem Querschnitt im ungequetschten Zustand der Leitung (4510) ist, und
- einen zweiten Abschnitt (452), der eine Leitung (4520) aufweist, die ein Ende aufweist, das mit dem Dialysatabgabesystem (51) verbunden ist, und die an einem gegenüberliegenden Ende mit einer Verbindungsvorrichtung (4521) versehen ist, die trennbar mit der zweiten Verbindungsvorrichtung (4512) des ersten Abschnitts (451) verbunden ist, um eine Verbindung in Reihe des ersten Abschnitts (451) und des zweiten Abschnitts (452) bereitzustellen, während es möglich ist, den ersten Abschnitt (451) vom zweiten Abschnitt (452) zu trennen, wobei die Leitung (4520) des zweiten Abschnitts (452) eine flexible Leitung mit konstantem Querschnitt im nicht gequetschten Zustand der Leitung (4520) ist,
so dass im getrennten und vom ersten Abschnitt (451) in Bezug auf den zweiten Abschnitt (452) abgezogenen Zustand die Verbindungsvorrichtung (4521) des zweiten Abschnitts (452) mit einem Verbinder (415') einer zweiten Dialysatversorgungsquelle (40') verbindbar und von dem Verbinder (415') der zweiten Dialysatversorgungsquelle (40') trennbar ist.

2. Maschine nach Anspruch 1, wobei das Verbindungssystem ein Verschlusselement (C451) umfasst, das gestattet, im geschlossenen Zustand die Zirkulation von Flüssigkeit im Inneren der Leitung (4510) des ersten Abschnitts (451) zu verhindern.

3. Maschine nach Anspruch 2, wobei das Verschlusselement (C451) vom manipulationssicheren Typ ist, so dass im geschlossenen Zustand das Verschlusselement (C451) verriegelt ist und/oder die Leitung (4510) des ersten Abschnitts (451) beeinträchtigt, um die Wiederverwendung der Leitung zu verhindern, nachdem das Verschlusselement (C451) geschlossen wurde.

4. Maschine nach einem der vorhergehenden Ansprüche, wobei der zweite Abschnitt (452) ein aktivierbares und deaktivierbares Öffnungs- und Verschlusselement (C452) umfasst, das erlaubt, die Zirkulation von Flüssigkeit durch die Leitung des zweiten Abschnitts (452) zu gestatten oder zu verhindern.

5. Maschine nach einem der vorhergehenden Ansprüche, wobei die erste Verbindungsvorrichtung (4511) des ersten Abschnitts (451) umfasst:
- ein röhrenförmiges Element (4511a), das imstande ist, in eine Öffnung des entsprechenden Verbinders (415) der ersten Dialysatversorgungsquelle (40) eingesetzt zu werden, um eine Kommunikation von Flüssigkeit im Inneren des röhrenförmigen Elements (4511a) zu gestatten; und
- eine Umfangswand (4511b), die sich um das röhrenförmige Element (4511a) herum und von diesem beabstandet erstreckt, um einen ringförmigen Raum zum Einsetzen einer Umfangswand des Verbinders der ersten Dialysatversorgungsquelle zu definieren.

6. Maschine nach einem der vorhergehenden Ansprüche, wobei die Verbindungsvorrichtung (4521) des zweiten Abschnitts (452) umfasst:
- ein röhrenförmiges Element (4521a), das imstande ist, in eine Öffnung der zweiten Verbindungsvorrichtung (4512) des ersten Abschnitts (451) eingesetzt zu werden, um eine Kommunikation von Flüssigkeit im Inneren des röhrenförmigen Elements (4521a) zu gestatten, und das im abgezogenen Zustand des ersten Abschnitts (451) imstande ist, in eine Öffnung des Verbinders (415') der zweiten Dialysatversorgungsquelle (40') eingesetzt zu werden, um eine Kommunikation von Flüssigkeit im Inneren des röhrenförmigen Elements (4521a) zu gestatten; und
- eine Umfangswand (4521b), die sich um das röhrenförmige Element (4521a) herum und von diesem beabstandet erstreckt, um einen ringförmigen Raum zum Einsetzen einer Umfangswand des zweiten Verbindungsvorrichtung (4512) des ersten Abschnitts (4521) zu definieren, oder um, im abgezogenen Zustand des ersten Abschnitts (451) einen ringförmigen Raum zum Einsetzen einer Umfangswand des Verbinders (415') der zweiten Dialysatversorgungsquelle (40') zu definieren.

7. Maschine nach einem der vorhergehenden Ansprüche, wobei die zweite Verbindungsvorrichtung (4512) des ersten Abschnitts (451) eine Umfangswand umfasst, die mit einem Außengewinde (4512a) versehen ist, das imstande ist, mit einem in der Umfangswand (4521b) der Verbindungsvorrichtung (4521) des zweiten Abschnitts (452) ausgebildeten Innengewinde zusammenzuwirken.

8. Maschine nach einem der vorhergehenden Ansprüche, wobei die Maschine umfasst:
- eine Dialysat-Zuführleitung (52), die mit einem Einlass (401) eines Dialysatdurchlassbereichs (14) des Dialysators (100) verbindbar ist, und
- eine Dialysat-Abführleitung (62), die mit einem Auslass (402) des Dialysatdurchgangsbereichs (14) des Dialysators (100) verbindbar ist.

9. Maschine nach Anspruch 8, wobei die Abführleitung (62) eine Leitung (6100) zum Verbinden mit einer Ableitungsvorrichtung wie ein Abwassersystem (Egt) und/oder ein System zur Rückgewinnung von gebrauchtem Dialysat umfasst.

10. Maschine nach einem der vorhergehenden Ansprüche, wobei die Maschine mindestens einen flexiblen Beutel (50), bezeichnet als Ventrikelbeutel, umfasst, der bestimmt ist, Dialysat zu enthalten, der einen Teil des Abgabesystems (51) bildet.

11. Maschine nach Anspruch 10, wobei der mindestens eine Beutel in einer Vorrichtung, bezeichnet als Kassette (9), enthalten ist, die in eine entsprechende Aufnahme einsetzbar und aus dieser entnehmbar ist, die in der Dialysemaschine eingerichtet ist.

12. Dialysemaschine nach Anspruch 11, wobei die Maschine einen Rahmen umfasst und die als Kassette bezeichnete Vorrichtung in Bezug auf den Rahmen der Maschine lösbar ist.

13. Verfahren zum sukzessiven Verbinden von Dialysatversorgungsquellen (40, 40') mit dem Verbindungssystem (450) einer Maschine nach einem der Ansprüche 1 bis 12, wobei das Verfahren die folgenden Schritte umfasst:
- Bereitstellen des Verbindungssystems (450) in einer Konfiguration, in der der erste Abschnitt (451) mit dem zweiten Abschnitt (452) verbunden ist, wobei der zweite Abschnitt an seinem anderen Ende mit dem Abgabesystem (51) für Dialysat (5) verbunden ist;
- Bereitstellen einer ersten Dialysatversorgungsquelle (40), die einen Verbinder (415) aufweist, der nicht mit dem Verbindungssystem (450) verbunden ist;
- wenn ein Stopfen (4519) das Ende des ersten Abschnitts (451) verschließt, das dem mit dem zweiten Abschnitt (452) verbundenen Ende gegenüberliegt, Entfernen des Stopfens (4519);
- Verbinden der ersten Verbindungsvorrichtung (4511) des ersten Abschnitts (451) mit der ersten Dialysatversorgungsquelle (40), und nach Verwendung der ersten Dialysatversorgungsquelle (40):
- vorzugsweise Verschließen des ersten Abschnitts (451), beispielsweise durch Verschließen eines Klemmsystems (C451) auf dem ersten Abschnitt,
- vorzugsweise Entfernen der ersten Versorgungsquelle (40) und Verschließen der Verbindungsvorrichtung (4511) des ersten Abschnitts (451) mit einem Stopfen (4519);
- Abziehen des ersten Abschnitts (451) in Bezug auf den zweiten Abschnitt (452) von der Vorrichtung, so dass der zweite Abschnitt (452) eine freie Verbindungsvorrichtung (4521) aufweist;
- Bereitstellen einer zweiten Dialysatversorgungsquelle (40') mit einem Verbinder (415'), der nicht mit dem Verbindungssystem (450) verbunden ist;
- Verbinden des Verbinders (415') der zweiten Dialysatversorgungsquelle (40') mit der freien Verbindungsvorrichtung (4521) des zweiten Abschnitts (452).

## Claims

1. A dialysis machine for treating a body fluid, such as blood or plasma, the dialysis machine comprising:
- a dialyzer (100, 100') comprising an enclosure which includes a body fluid passage zone (12) which has a body fluid inlet (201) and a body fluid outlet (202), and a dialysate passage zone (14) which has a dialysate inlet (401) and a dialysate outlet (402), and a membrane system (3) between the body fluid passage zone and the dialysate passage zone (14);
- a dialysate delivery system (51),
- a connection system (450) for connecting a dialysate supply source (40) to the dialysate delivery system (51), the connection system (450) comprising:
- a first section (451), which comprises a conduit (4510) provided at one end with a first connection device (4511) which is connectable to a connector (415) of a first dialysate supply source (40), and which is disconnectable from said connector (415) of the first dialysate supply source (40), and provided at the other end with a second connection device (4512), said conduit (4510) being a flexible conduit of constant cross section in the uncrushed state of said conduit (4510), and
- a second section (452), which comprises a conduit (4520) which has one end connected to the dialysate delivery system (51) and which is provided at an opposite end with a connection device (4521) which is connected, in a disconnectable manner, to the second connection device (4512) of the first section (451), in order to provide a series connection of the first section (451) and the second section (452), while allowing said first section (451) to be disconnected from the second section (452), said conduit (4520) of the second section (452) being a flexible conduit of constant cross section in the uncrushed state of said conduit (4520),
so that, in the state with the first section (451) disconnected and removed from the second section (452), said connection device (4521) of the second section (452) is connectable to a connector (415') of a second dialysate supply source (40') and disconnectable from said connector (415') of the second dialysate supply source (40').

2. The machine as claimed in claim 1, wherein the connection system comprises a closing element (C451) which, in the closed state, makes it possible to prevent liquid from circulating inside the conduit (4510) of the first section (451).

3. The machine as claimed in claim 2, wherein the closing element (C451) is of the tamper-evident type so that, in the closed state, the closing element (C451) is locked and/or damages the conduit (4510) of the first section (451) in order to prevent reuse of the conduit after the closing element (C451) has been closed.

4. The machine as claimed in any one of the preceding claims, wherein the second section (452) comprises an activatable and deactivatable opening and closing element (C452) making it possible to allow or prevent the circulation of liquid through the conduit of the second section (452).

5. The machine as claimed in any one of the preceding claims, wherein the first connection device (4511) of the first section (451) comprises:
- a tubular element (4511a) capable of being engaged in an opening of the corresponding connector (415) of the first dialysate supply source (40) in order to allow liquid communication inside said tubular element (4511a); and
- a peripheral wall (4511b) which extends around and at a distance from the tubular element (4511a) in order to define an annular space for insertion of a peripheral wall of said connector of the first dialysate supply source.

6. The machine as claimed in any one of the preceding claims, wherein the connection device (4521) of the second section (452) comprises:
- a tubular element (4521a) which is capable of being engaged in an opening of the second connection device (4512) of the first section (451) in order to allow liquid communication inside said tubular element (4521a), and which, in the withdrawn state of the first section (451), is capable of being engaged in an opening of the connector (415') of the second dialysate supply source (40') in order to allow liquid communication inside said tubular member (4521a); and
- a peripheral wall (4521b) which extends around and at a distance from the tubular element (4521a) in order to define an annular space for insertion of a peripheral wall of said second connection device (4512) of the first section (4521) or, in the withdrawn state of the first section (451), to define an annular space for insertion of a peripheral wall of the connector (415') of the second dialysate supply source (40').

7. The machine as claimed in any one of the preceding claims, wherein the second connection device (4512) of the first section (451) comprises a peripheral wall provided with a thread (4512a) able to cooperate with an internal thread formed in the peripheral wall (4521b) of the connection device (4521) of the second section (452).

8. The machine as claimed in any one of the preceding claims, wherein the machine comprises:
- a dialysate feed line (52) which is connectable to an inlet (401) of a dialysate passage zone (14) of the dialyzer (100), and
- a dialysate discharge line (62) which is connectable to an outlet (402) of the dialysate passage zone (14) of the dialyzer (100).

9. The machine as claimed in claim 8, wherein the discharge line (62) comprises a conduit (6100) for connection to a discharge device such as a drainage system (Egt) and/or a spent dialysate recovery system.

10. The machine as claimed in any one of the preceding claims, wherein the machine comprises at least one flexible bag (50), called a ventricle bag, intended to contain dialysate and forming part of said delivery system (51).

11. The machine as claimed in claim 10, wherein said at least one bag is included in a device, called a cassette (9), which is insertable into and removable from a corresponding housing provided in the dialysis machine.

12. The dialysis machine as claimed in claim 11, wherein the machine comprises a frame and said device, called a cassette, is removable from the frame of the machine.

13. A method for successively connecting dialysate supply sources (40, 40') to the connection system (450) of the machine as claimed in any one of claims 1 to 12, the method comprising the following steps:
- providing the connection system (450) in a configuration in which the first section (451) is connected to the second section (452), the second section being connected by its other end to the dialysate (5) delivery system (51);
- providing a first dialysate supply source (40) having a connector (415) not connected to the connection system (450);
- when a stopper (4519) closes the end of the first section (451) opposite the end connected to the second section (452), removing said stopper (4519);
- connecting the first connection device (4511) of the first section (451) to the first dialysate supply source (40), and, after use of the first dialysate supply source (40):
- preferably closing off the first section (451), for example by closure of a clamp system (C451) on the first section,
- preferably removing the first dialysate supply source (40) and closing the connection device (4511) of the first section (451) with the aid of a stopper (4519);
- removing the first section (451) from the second section (452) of the device so that the second section (452) has a free connection device (4521);
- providing a second dialysate supply source (40') having a connector (415') not connected to the connection system (450);
- connecting the connector (415') of the second dialysate supply source (40') to the free connection device (4521) of said second section (452).
